# EUROPEAN PATENT SPECIFICATION

(11) **EP 3 511 905 B1**
(45) Date of publication and mention of the grant of the patent: **10.11.2021**
(21) Application number: 19155949.1
(22) Date of filing: 19.12.2016
(51) Int. Cl.: G06T 7/73, G06T 7/00

(54) **SYSTEMS AND METHODS FOR INTRA-OPERATIVE IMAGE ANALYSIS**
SYSTEME UND VERFAHREN ZUR INTRAOPERATIVEN BILDANALYSE
SYSTÈMES ET PROCÉDÉS D'ANALYSE D'IMAGES INTRA-OPÉRATOIRES

(30) Priority: 18.12.2015 US 201514974225
(43) Date of publication of application: 17.07.2019
(62) Divisional of application: 16876926.3
(73) Proprietor: DePuy Synthes Products, Inc., Raynham, MA 02767 (US)
(72) Inventor: WOLLOWICK, Noah, D., Westport, CT 06880 (US); COOPER, Andrew, J., Largo, FL 33770 (US)
(74) Representative: Carpmaels & Ransford LLP

(56) References cited:
- WO-A1-2007/009263
- US-A1- 2015 238 271
- Anonymous: "S U R G I C A L T E C H N I Q U E", , 1 January 2005 (2005-01-01), XP055578396, Retrieved from the Internet: URL:http://www.bizwan.com/_mydoc/depuy/hip /Corail%20Surgical%20Technique%200612-82-5 01.pdf [retrieved on 2019-04-08]

## Description

### FIELD OF THE INVENTION

The invention relates to analysis of images of features within a patient and more particularly to accurately analyzing such images during surgery.

### BACKGROUND OF THE INVENTION

There are a variety of intraoperative systems that assist orthopaedic surgeons in determining leg length and offset when performing hip arthroplasty. Many techniques utilize a "trial" prosthetic device as an initial implant.

While existing systems provide intraoperative leg length and offset data, they do not predict how changing the modular options in a prosthetic system will affect leg length and offset. When an inserted prosthetic has not created the desired leg length and offset, the surgeon is forced to repeat a process that involves dislocating the hip, replacing the original trial prosthetic with another prosthetic option that they think might provide a closer leg length and offset data to what they are trying to achieve, and then repeat a process of analyzing leg length and offset data using their respective guidance systems. Without guidance that predicts how changing modular prosthetic options would affect leg length and offset, the surgeon may repeat this trialing process multiple times.

This process of re-trialing is time-consuming for the surgeon, and increases the risk of femoral fractures due to the repetitious process of dislocating the hip to change the modular prosthetic selection. A system that could eliminate the need for progressive trialing to optimize leg length and offset, along with the associated time and risks, would benefit both the patient and surgeon when performing hip arthoplasty.

For the foregoing reasons, there is a need for an intraoperative guidance system that can predict intraoperative leg length and offset changes without requiring re-trialing to update modular system options.

US2015238271A1 discusses a system and method for analyzing images to optimize orthopaedic functionality at a site within a patient, including obtaining at least a first, reference image of the site, or a corresponding contralateral site, the first image including at least a first anatomical region or a corresponding anatomical region. At least a second, intraoperative results image of the site is obtained.

### SUMMARY OF THE INVENTION

This invention results from the realization that offset and length differential of an implant having at least one center of rotation can be accurately estimated during surgery by establishing at least one stationary point on the skeletal bone and at the center of rotation in an intraoperative image, aligning a digital implant representation with the implant, and then copying and positioning the digital representation in at least one reference image including one of (a) a preoperative image of the surgical site and (b) a contralateral image on an opposite side of the patient from the surgical site. Another realization is that changes in offset and length differential can be estimated based on selected alternative changes in at least one dimension of the implant for potential alternative implants.

This invention features a system and method that acquire (i) at least one reference image including one of a preoperative image of a surgical site with skeletal and articulating bones and a contralateral image on an opposite side of the patient from the surgical site, and (ii) at least an intraoperative image of the site after an implant has been affixed to the articulating bone. The system and/or method generates at least one reference stationary point on at least the skeletal bone in the reference image and at least one intraoperative stationary point on at least the skeletal bone in the intraoperative image, such as a tear drop or other feature associated with a pelvic bone of a patient. The location of the implant is identified in the intraoperative image, including the position of first and second centers of rotation, which are co-located in the intraoperative image. At least a first digital implant representation is aligned with the skeletal component and with at least the intraoperative stationary point, and at least a second digital implant representation is aligned with the articulating bone component and at least one point, such as a landmark point or a digital point annotation on the greater trochanter of a femur, on the articulating bone. The digital representations are copied and positioned in the reference image in an equivalent location relative to at least the reference stationary point and the articulating bone to determine the position of the first and second centers of rotation relative to each other in the reference image. Any differences between the locations of the first and second centers of rotation in the reference image are utilized to analyze at least one of offset and length differential.

In one system embodiment, the system includes a memory, a user interface including a display capable of providing at least visual guidance to a user of the system, and a processor, with the processor executing a program performing at least the steps listed above and described in more detail below. In some embodiments for the system and/or method, analyzing includes generating a vector having its origin at the reference stationary point and its terminal point at the first center of rotation. In certain embodiments, identifying includes determining a longitudinal axis for the second digital implant representation and analyzing includes utilizing a difference in spacing (i) perpendicular to the longitudinal axis to calculate offset and (ii) parallel to the longitudinal axis to calculate length differential. In one embodiment, the pelvis of the patient is selected as the skeletal bone and a femur is selected as the articulating bone, and the skeletal component of the implant is an acetabular cup and the articulating bone component includes a femoral stem having a shoulder, and the reference stationary point and the intraoperative stationary point are generated to have a known location relative to an obturator foramen of the patient, such as the tear drop. In one embodiment, the point on the articulating bone is identified to have a known location relative to the greater trochanter on the femur of the patient, such as the point being positioned on the tip of the greater trochanter.

In certain embodiments, the system and method further include generating at least one of offset and length differential for an assortment of implant components, such as a variety of modular implant options for different stem offset, lengths and/or head size. The assortment may include a listing of available size and length options for different implant components, and the system or method then provides at least one of (1) a recommendation of ideal or optimum implant selections and (2) a visually-perceptible chart or table of at least one of offset and length differential for each available implant option.

This invention also features a system to analyze images at a surgical site within a patient, the surgical site including at least a first, skeletal bone and a second, articulating bone that articulates with the skeletal bone at a joint, the system including an image selection module capable of acquiring (i) at least a first, reference image including one of a preoperative image of the surgical site and a contralateral image on an opposite side of the patient from the surgical site, and (ii) at least a second, intraoperative image of the site after an implant has been affixed to the articulating bone. The implant has at least a skeletal component with a first center of rotation and an articulating bone component having a second center of rotation, the first and second centers of rotation being co-located in the intraoperative image. The system optionally includes a digital annotation module, also referred to as a landmark identification module, capable of receiving the reference and intraoperative images and generating at least one reference stationary point on at least the skeletal bone in the reference image and at least one intraoperative stationary point on at least the skeletal bone in the intraoperative image. A templating module is capable of (a) identifying the location of the implant in the intraoperative image, including the position of the first and second centers of rotation, and aligning (i) at least a first digital implant representation with the skeletal component and with at least the intraoperative stationary point, and (ii) at least a second digital implant representation with the articulating bone component and at least one point on the articulating bone, and (b) copying the first and second digital representations and positioning them in the reference image in an equivalent location relative to at least the reference stationary point and the articulating bone to determine the position of the first and second centers of rotation relative to each other in the reference image. A calculation module, also referred to as an analysis module, is capable of utilizing any differences between the locations of the first and second centers of rotation in the reference image to analyze at least one of offset and length differential of at least one of the articulating bone and the implant in the intraoperative image.

In some embodiments, the reference and intraoperative images are provided by the image selection module to the data input module in a digitized format. In certain embodiments, the templating module positions the first digital representation in the reference image relative to the reference stationary point according to at least an intraoperative vector calculation utilizing at least the intraoperative stationary point relative to the first center of rotation and a reference vector calculation utilizing at least the reference stationary point relative to the first center of rotation. In one embodiment, the reference vector calculation replicates the intraoperative vector calculation. In a number of embodiments, the digital annotation module further generates at least a reference digital point annotation or reference landmark point on at least one anatomical feature on the articulating bone in the reference image and at least an intraoperative digital point annotation or landmark point on at least that anatomical feature on the articulating bone in the intraoperative image and, in one embodiment, at least one of the templating module and the calculation module utilizes the digital annotation or landmark points to assist alignment of the second digital implant representation on the articulating bone in both of the reference and intraoperative images.

In certain embodiments, the templating module selects a fixed point on the second digital implant representation and the calculation module is capable of estimating changes in offset and length differential based on selected alternative changes in at least one dimension of the implant for alternative implants, each with a similar fixed point, to be considered by a user of the system as a replacement for the implant in the intraoperative image. In some embodiments, the reference image and the intraoperative image are at least one of rotated, aligned and scaled relative to each other prior to the templating module copying the digital representation and positioning it in the reference image. In one embodiment, the digital annotation module generates at least one other stationary point on the skeletal bone in the reference image to establish a reference stationary base and at least one other stationary point on the skeletal bone in the intraoperative image to establish an intraoperative stationary base, and the calculation module utilizes the reference and intraoperative stationary bases to accomplish at least one of image rotation, image alignment and image scaling. In another embodiment, the calculation module provides at least relative scaling of one of the reference and intraoperative images to match the scaling of the other of the reference and intraoperative images. In yet another embodiment, the calculation module utilizes at least one object of known dimension in at least one of the reference and intraoperative images to provide absolute scaling to at least that image.

This invention further features a system analyze images at a surgical site within a patient, the surgical site including at least a first, skeletal bone and a second, articulating bone that articulates with the skeletal bone at a joint, the system including an image selection module capable of acquiring (i) at least one digitized reference image including one of a preoperative image of the surgical site and a contralateral image on an opposite side of the patient from the surgical site, and (ii) at least one digitized intraoperative image of the site after an implant has been affixed to the articulating bone, the implant having at least a skeletal component with a first center of rotation and an articulating bone component having a second center of rotation, the first and second centers of rotation being co-located in the intraoperative image. The system also includes a templating module capable of (a) identifying the location of the implant in the intraoperative image and aligning at least one of (i) at least a first digital implant representation with the skeletal component and with at least one intraoperative stationary point on at least the skeletal bone, and (ii) at least a second digital implant representation with the articulating bone component and at least one point on the articulating bone, and (b) copying at least one of the first and second digital representations and positioning them in the reference image in an equivalent location relative to at least one of (A) a reference stationary point on at least the skeletal bone and (B) at least one reference point on the articulating bone, respectively, in the reference image. The system further includes an calculation module capable of utilizing any differences between the locations of at least one of the first and second digital implant representations in the reference image to analyze at least one of offset and length differential of at least one of the articulating bone and the implant in the intraoperative image. The templating module selects a fixed point on the second digital implant representation and the calculation module is capable of estimating changes in offset and length differential based on selected alternative changes in at least one dimension of the implant for alternative implants, each with a similar fixed point, to be considered by a user of the system as a replacement for the implant in the intraoperative image.

In one embodiment, the system further includes a digital annotation module capable of receiving the reference and intraoperative images and generating the at least one reference stationary point on at least the skeletal bone in the reference image and the at least one intraoperative stationary point on at least the skeletal bone in the intraoperative image.

This invention still further features a method for analyzing images to optimize the restoration of orthopaedic functionality at a surgical site within a patient, the surgical site including at least a first, skeletal bone and a second, articulating bone that articulates with the skeletal bone at a joint, the method including the steps of acquiring (i) at least one digitized reference image including one of a preoperative image of the surgical site and a contralateral image on an opposite side of the patient from the surgical site, and (ii) at least one digitized intraoperative image of the site after an implant has been affixed to the articulating bone, the implant having at least a skeletal component with a first center of rotation and an articulating bone component having a second center of rotation, the first and second centers of rotation being co-located in the intraoperative image. The method includes identifying the location of the implant in the intraoperative image and aligning at least one of (i) at least a first digital implant representation with the skeletal component and with at least one intraoperative stationary point on at least the skeletal bone, and (ii) at least a second digital implant representation with the articulating bone component and at least one point on the articulating bone. At least one of the first and second digital representations are copied and positioned in the reference image in an equivalent location relative to at least one of (A) a reference stationary point on at least the skeletal bone and (B) the articulating bone, respectively, in the reference image. Any differences between the locations of at least one of the first and second centers of rotation in the reference image are utilized to analyze at least one of offset and length differential of at least one of the articulating bone and the implant in the intraoperative image. A fixed point on the second digital implant representation is selected, and changes in offset and length differential are estimated based on selected alternative changes in at least one dimension of the implant for alternative implants, each with a similar fixed point, to be considered by a user of the system as a replacement for the implant in the intraoperative image.

### BRIEF DESCRIPTION OF THE DRAWINGS

In what follows, preferred embodiments of the invention are explained in more detail with reference to the drawings, in which:
FIGS. 1A-1B are flowcharts of a OneTrial™ analysis system according to the present invention calculating leg length and offset for all known modular head and stem offset combinations of a particular stem model and size;
FIG. 2 is a schematic diagram of the OneTrial™ System according to the present invention;
FIG. 3 is a schematic screen view of a preoperative radiographic image of a patient's left hip;
FIG. 4 is a schematic screen view of a digital point annotation placed on the greater trochanter in the preoperative hip image;
FIG. 5 is a schematic screen view with a digital circle annotation placed around the femoral head in the preoperative hip image;
FIG. 6 is a schematic screen view of a digital annotation placed along the longitudinal axis of the femur in the preoperative hip image;
FIG. 7 is a schematic screen view of a digital line annotation drawn between two identifiable points on the pelvic anatomy of the preoperative hip image;
FIG. 8 is a schematic screen view of a digital point annotation placed on the pelvic teardrop in the preoperative hip image;
FIG. 9 is a schematic screen view of a screen that asks the user if they want to proceed to or skip acetabular component position analysis;
FIG. 10 is a schematic screen view of a screen that guides the user in acquiring an image of the operative hip;
FIG. 11 is a schematic screen view guiding the user to place a digital point annotation on the greater trochanter on the operative hip image;
FIG. 12 is a schematic screen view guiding the user to place a digital circle annotation around the acetabular component and enter the component size on the operative hip image;
FIG. 13 is a schematic screen view guiding the user to draw a digital line annotation between two identifiable point on the pelvic anatomy in the operative hip image;
FIG. 14 is a schematic screen view used to enter the specifications of the acetabular component inserted into the operative hip;
FIG. 15 is a schematic screen view enabling the user to position the acetabular component template so that it matches the position of the acetabular component in the operative hip image;
FIG. 16 is a schematic screen view used to enter the specifications of the femoral trial stem inserted into the operative hip;
FIG. 17 is a schematic screen view enabling the user to position the femoral stem template so that it matches the position of the trial femoral stem component in the operative hip image;
FIG. 18 is a schematic screen view of a section of the operative hip from the intraoperative image overlaid on the preoperative hip image;

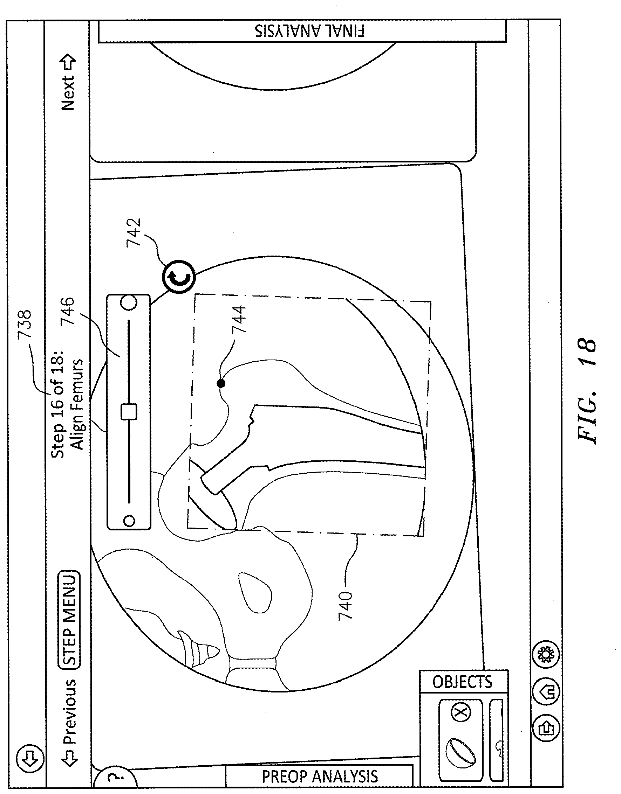

FIG. 18 is a schematic screen view of a section of the operative hip from the intraoperative image overlaid on the preoperative hip image; and

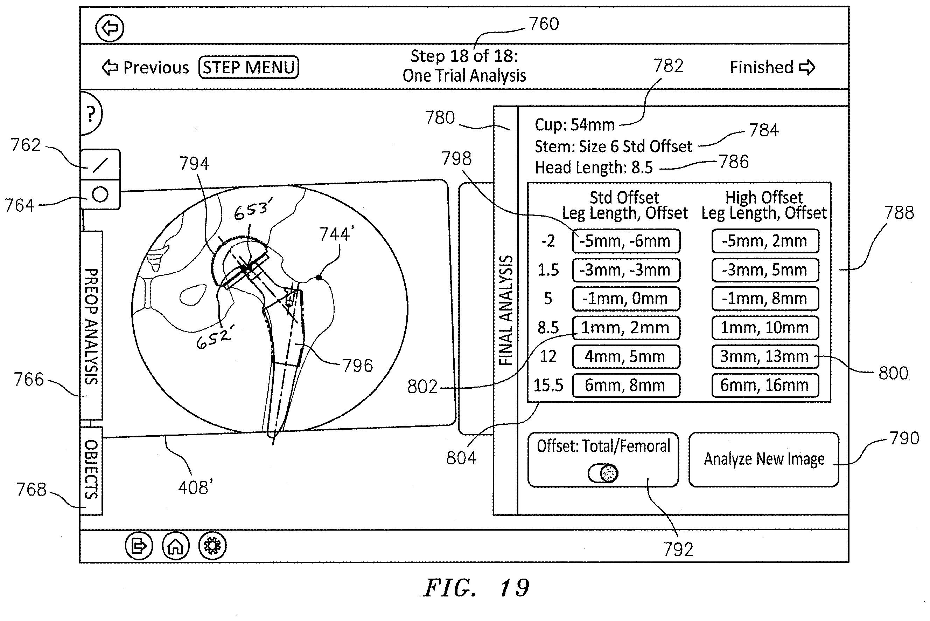

FIG. 19 is a schematic screen view of a preoperative image, with digital templates superimposed, on the left and a final analysis chart of leg length and offset data for all stem offset and head combinations for the inserted trial prosthetic.

### DETAILED DESCRIPTION OF THE PRESENTLY PREFERRED EMBODIMENTS

This invention may be accomplished by a system and method that acquire (i) at least one reference image including one of a preoperative image of a surgical site with skeletal and articulating bones and a contralateral image on an opposite side of the patient from the surgical site, and (ii) at least one intraoperative image of the site after an implant has been affixed to the articulating bone. In certain constructions, the system generates at least one reference stationary point on at least the skeletal bone in the reference image and at least one intraoperative stationary point on at least the skeletal bone in the intraoperative image. The location of the implant is identified in the intraoperative image, preferably including the position of first and second centers of rotation which are co-located in the intraoperative image. At least one of (i) a first digital implant representation is aligned with the skeletal component and with at least the intraoperative stationary point, and (ii) a second digital implant representation is aligned with the articulating bone component and at least one point on the articulating bone. One or more of the digital representations are copied and positioned in the reference image in an equivalent location relative to at least one of the reference stationary point and the articulating bone to directly or indirectly determine the position of the first and second centers of rotation relative to each other in the reference image. Any differences between the locations of at least one of the first and second centers of rotation in the reference image are utilized to analyze at least one of offset and length differential. Finally, the system will generate at least one of length and offset differential data for a range of implant options using data associated with the digital implant representations.

The term "digital representation" or "digital implant representation" as utilized herein includes a digital template or other digital annotation, such as a digital line having at least two points, e.g. a line representing a longitudinal axis or a diameter of an implant or a bone, or a digital circle or other geometric shape which can be aligned with an implant or a bone intraoperatively and then placed in a corresponding location in a preoperative image.

Software to accomplish the techniques described herein is generally located on a single computing device, such as an iPad, Android tablet, or Microsoft Surface device, containing at least a processor, memory, and a viewing screen. Options for the user to interact with the computing device include touching the screen, using a stylus or similar object to interact with the device, voice commands, and use of a standard computer mouse that can receive user input by direct touch.

Image acquisition may be accomplished through a variety of techniques that include use of a camera to take a picture of an imaging screen and directly importing an image from another digital system. If image acquisition is performed using a camera, then a digital camera must either be contained within the computing device or otherwise connected to the computing device either directly or over a network. Communication between the computing device and a separate camera may be accomplished using a wireless protocol such as Bluetooth, a direct connection such as an Ethernet or USB cable, a TCP/IP internet connection, or similar technology. If image acquisition is performed by directly importing an image, the image may be imported from another system using a protocol such as DICOM or through a direct file upload. The image may alternatively be directly imported from a digital radiographic system such as a fluoroscopic c-arm, for example the General Electric OEC 9900 Elite system. To directly import an image from a c-arm such as this, an output port may interface directly with the computing device. Depending on the computing device used, an intermediate device will take the data from the output port, pre-process the data to make it compatible with the computing device, and then send the computing data to the computing device so that it can be used within the system.

This system can be used to guide surgical decision making during hip arthroplasty. In addition to providing leg length and offset data for the inserted prosthetic system, the system precisely calculates leg length and offset data for all modular options of a specific stem size and model before they are implanted in the body. These modular options may include stem offset and head selections, although the specific modular options that are available vary based upon the selected implant model. The generation of this data without trialing is an invaluable tool for a surgeon because it streamlines the process of final implant selection by eliminating the need for progressive trialing.

Traditionally, a surgeon may need to progressively trial multiple stems to select an optimal stem combination during surgery. This traditional trialing process requires the surgeon to dislocate the hip, change the implant selection, and then re-analyze leg length and offset data, either visually or through an alternative computer navigation system. The data provided by a OneTrial™ system according to the present invention provides the surgeon with the data to eliminate the time-consuming and higher-risk need for progressive trialing during surgery.

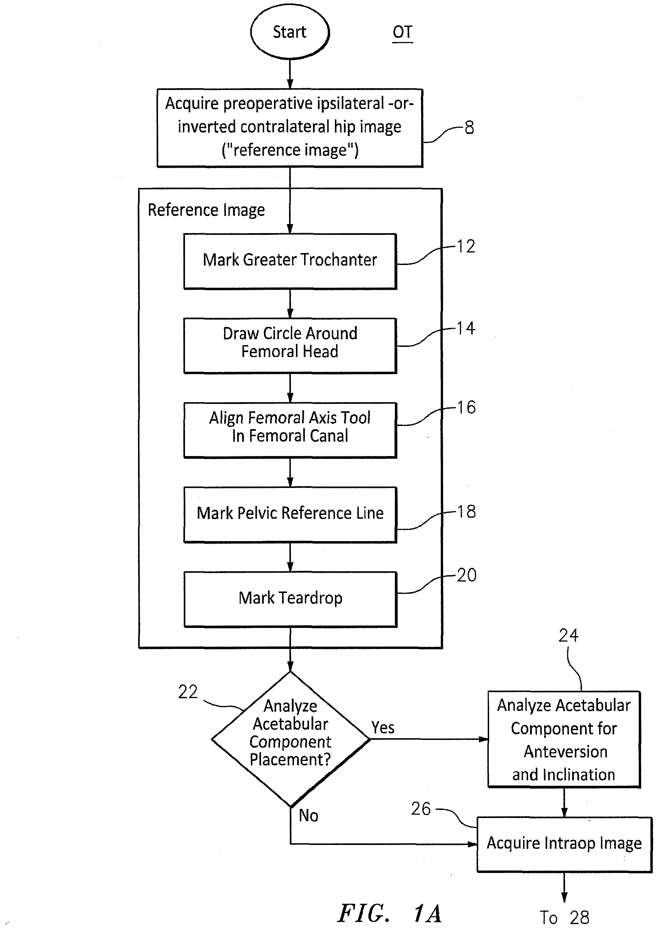

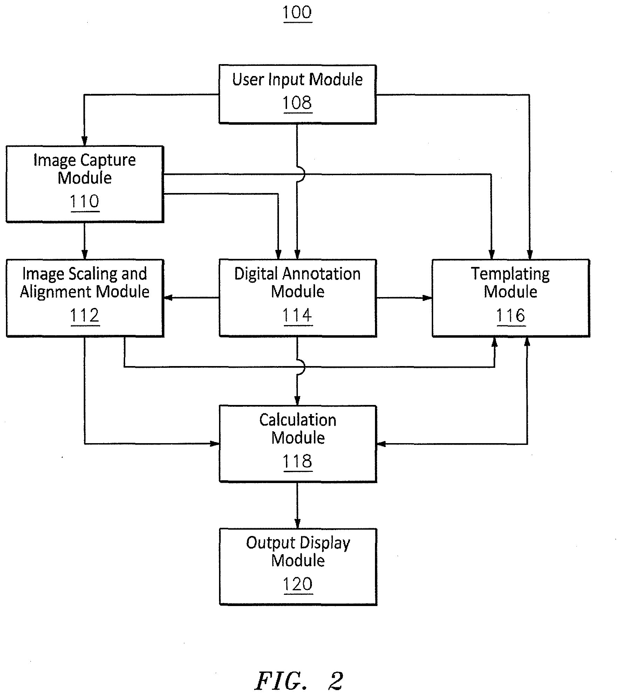

In one preferred construction of the OneTrial™ system, the process begins as shown in flowchart OT, FIGS. 1A-1B, by acquiring, step 8, FIG. 1A, either a selected preoperative ipsilateral image or a selected inverted contralateral image. Whichever image is selected is referred to herein as a "first, reference image" or "preop image". Image acquisition is initiated by Image Capture Module 110 of OneTrial™ system 100, FIG. 2, that allows the user to press a button-type icon within a medical software application to initiate the image upload process. The system may support one or more of various techniques to perform image acquisition. These techniques include file upload, DICOM integration from a radiographic of PACS system, or image direct import from a fluoroscopy system such as the General Electric OEC 9900 Elite. Use of a digital camera, connected to the system's computing device, to take a picture of a fluoroscopic or radiographic viewing screen may alternatively be used to acquire the preop image. If a digital camera is used to acquire an image, an effort should be made to ensure that a camera picture of the digital viewing screen is not obtained at an angle, as this could create distortion in the acquired preop image and adversely affect the data output by the system.

When a contralateral image is used as the 'preop' image, the OneTrial™ system may automatically flip the image within the software, so that it will match the orientation of the operative image. Alternatively, the contralateral image may be flipped using the c-arm fluoroscopy system prior to image acquisition.

After acquiring the preop image, the method continues with Digital Annotation Module 114, FIG. 2, placing a series of digital annotations on the preop image in steps 12, 14, 16, 18, and 20, FIG. 1A. In preferred constructions, the system will use automated image recognition and other known information, such as previously annotated anatomy, to automatically place the annotations on the preop image, after which the annotations can be modified by the user. The system makes use of similar data on the intraop image to automatically generate initial annotation placement before it can be modified by the user.

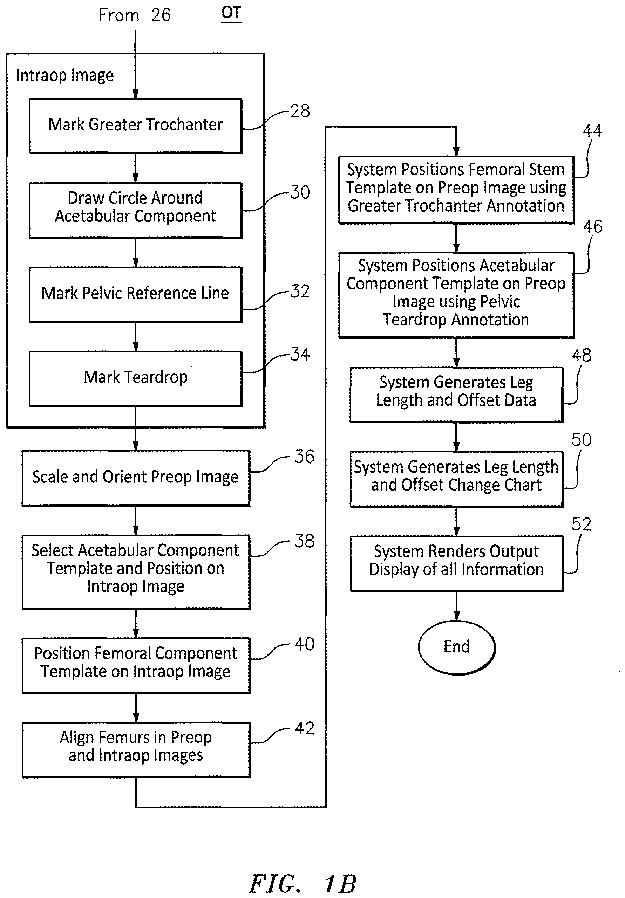

The series of preop annotation steps commences with Step 12, FIG. 1 in which Digital Annotation Module 114, FIG. 2 guides the placement of a digital point annotation that identifies the greater trochanter. While the system may use any identifiable point on the femur, the greater trochanter is an ideal point because the anatomy is less sensitive to rotation when using 2-d radiographic imaging compared to other identifiable points, such as the lesser trochanter.

In Step 14, FIG. 1A, Digital Annotation Module 114, FIG. 2, guides the placement of a digital circle annotation that approximates the center of rotation of the hip in the preop image.

The method continues with Step 16, FIG. 1, in which Digital Annotation Module 114, FIG. 2, guides the placement of an annotation that appears as the lower portion of a hip prosthetic, referred to as the 'femoral axis tool'. Specifically, the annotation is a digital rendering of a hip template with the neck and head of the prosthetic removed. The user can reposition the annotation so that it fully fills the femoral canal. This object is used to identify the location of the longitudinal axis of the femoral canal. This digital rendering annotation contains a line that continues along the longitudinal axis of the annotation, so that the location of the midpoint of the femur can be identified in the image.

Alternative constructions may use other annotations to identify the midpoint of the femoral canal. For example, the user may be prompted in alternative constructions to draw a digital line down the central axis of the femur, or else a rectangular object that can be positioned along the femoral cortices so that the central axis of the femur can be calculated mathematically. However, the preferred construction makes use of an annotation that looks like the distal portion of a hip prosthetic because it has been found to be more accurate in correctly determining the longitudinal axis of the femur, whereas alternative constructions have been found to have more error due to the mal-positioning in the wider area of the bone on the proximal femur.

In Step 18, FIG. 1A, Digital Annotation Module 114, FIG. 2, guides the placement of a digital line that connects two identifiable points on the stable pelvic anatomy. In a later step, this digital line will be drawn to connect the same anatomic points on the intraop images, and the system will then use these lines to match the rotational alignment, and possibly scaling, of the preop and intraop images based on pelvic anatomy.

In Step 20, FIG. 1A, Digital Annotation Module 114, FIG 2, guides the placement of a digital point annotation that identifies the teardrop point on the pelvis in the preop image. Step 20 is the final step in which an annotation (other than a digital template) is placed on the preop image.

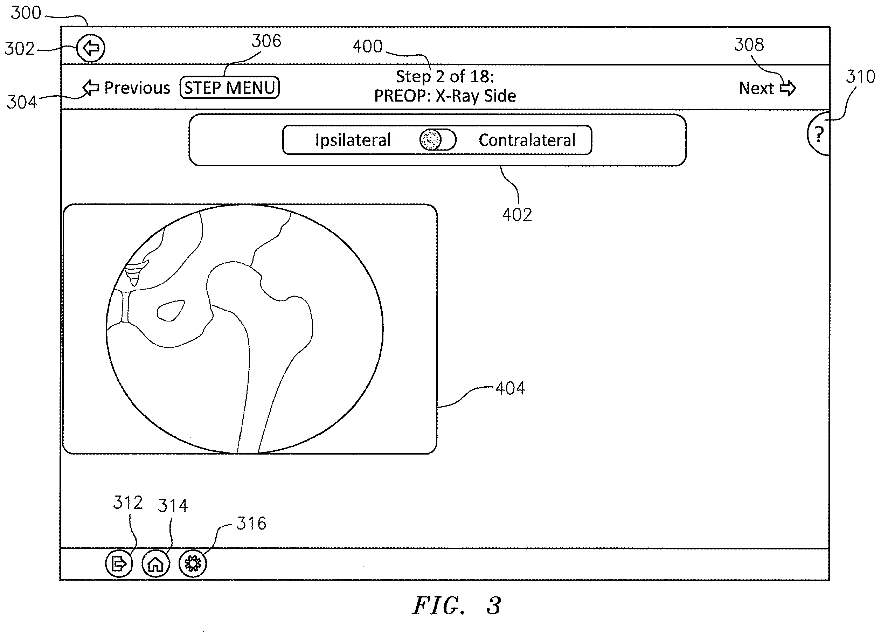

FIG. 3 is a schematic representation of a digital screen view 300 of a mobile computing device, such as an iPad, Android tablet, or Microsoft tablet. The screen view 300 shows a series of navigation objects that are used throughout the implementation of the system. Clickable icon 302 allows the user to exit the module in entirety to return to a home screen. Clickable button 304 brings the user to the previous step in the multi-step system, and clickable button 308 brings the user to the next step after completing the existing step. Selecting clickable button 306, "Step Menu", opens a digital menu that shows all steps associated with the system implementation, and allowing the user to return to any step previously done to review or make changes. Clickable icons 312, 314, and 316 at the bottom left of the screen allow the user to exit the application, return to the home screen, and open configuration settings, respectively. Clickable icon 310 opens a Help Panel which provides context-sensitive help that provides user guidance on how to use the system. The digital screen message 400 "Step 2 of 18: PREOP: X-Ray Side", provides the user with information on where they are in the system. Toggle 402 allows the user to tell the system whether the preop image being used is a preop ipsilateral image, or otherwise a contralateral image. Digital image 404 is a preop image, obtained from a fluoroscopy system in Step 8, FIG. 1A.

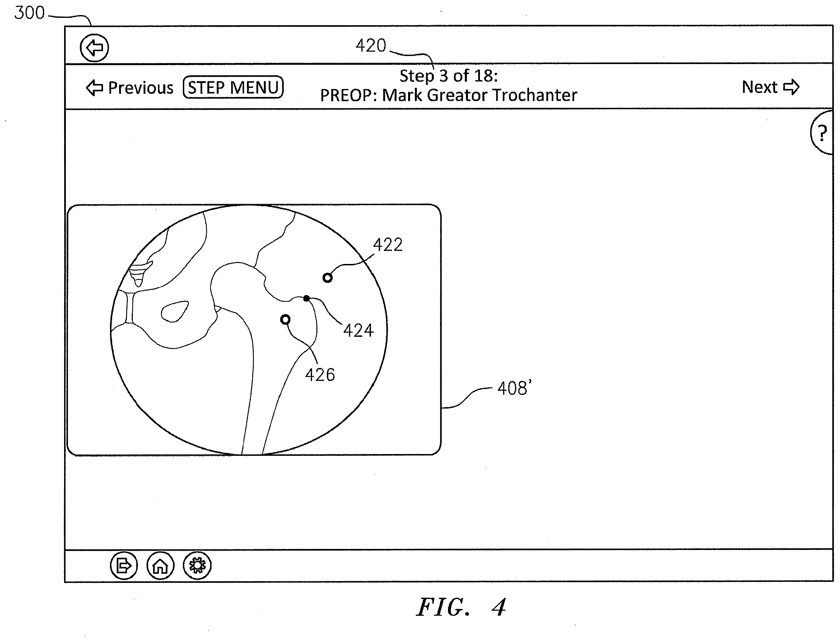

FIG. 4 shows the same digital screen view 300' with the same preop image 408'. Global icons 302', 304', 306', 308', 310', 312', 314', and 316' provide the same navigation functionality in this screen, and all other screens, as how they are described in FIG. 3. Digital screen message 420 "Step 3 of 18: PREOP: Mark Greater Trochanter" provides indication to the user that they are adding a digital annotation to the greater trochanter in this preop image, as shown in FIG. 1A, Step 12. Digital handles 422 and 426 allow the user to manipulate the location of digital point annotation 424, so that it is placed in a desired reproducible location on the greater trochanter.

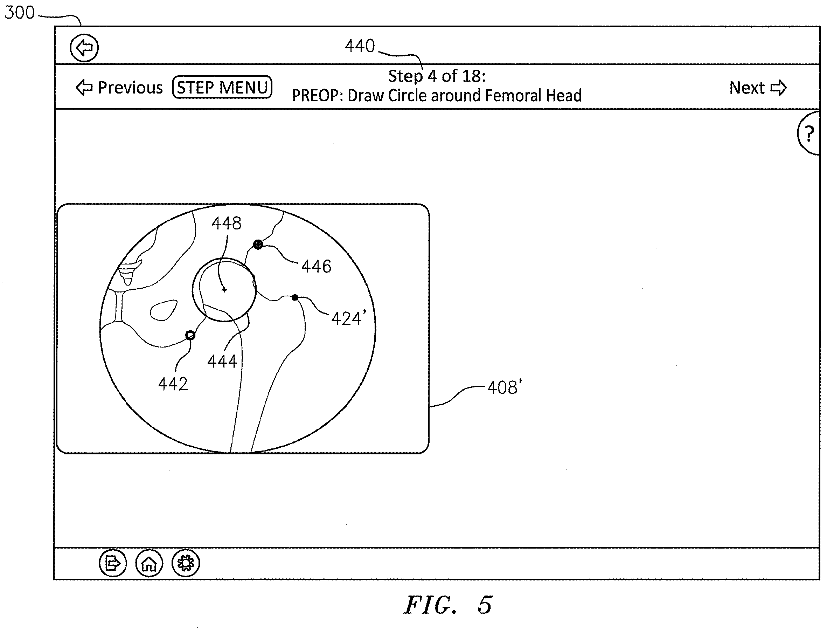

FIG. 5 shows the same digital screen view 300' with the same preop image 408'. Digital screen message 440 "Step 4 of 18: PREOP: Draw Circle Around Femoral Head" provides indication to the user that they are to position a digital circle annotation around the femoral head in preop image 408', as shown in FIG. 1A, Step 14. Digital handle 442 enables the user to manipulate the location of digital circle 444 with a displayed digital center point 448 so that it can be placed to identify the femoral head location. Digital handle 446 additionally allows the user to manipulate the size of the digital circle 444. Digital annotation 424' identifies the previously identified location of the greater trochanter. Preferred implementations will try to auto-identify an appropriate starting location for digital circle via the use of image recognition and known data of the greater trochanter 424', which was digitally annotated in a previous step.

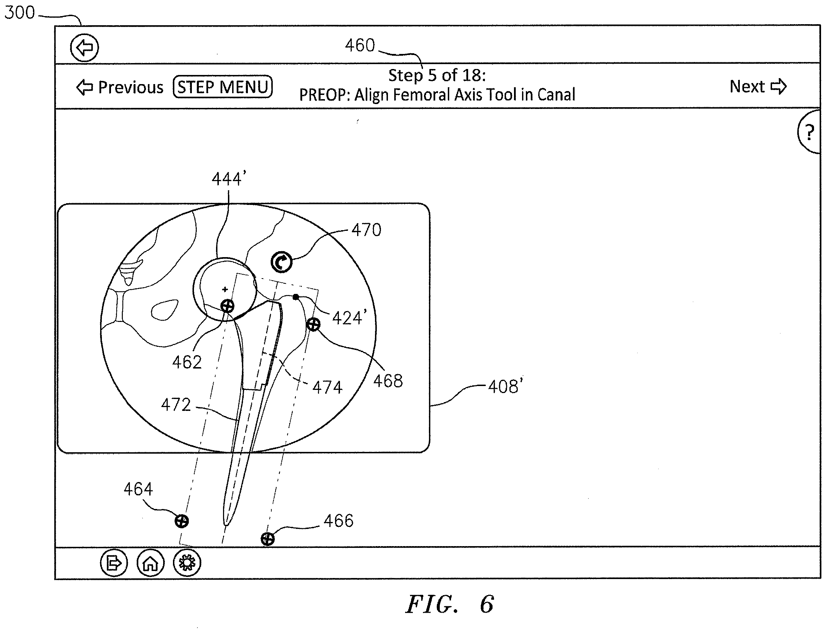

FIG. 6 shows digital screen view 300' with the same preop image 408'. Digital screen message 460 "Step 5 of 18: PREOP: Align Femoral Axis Tool in Canal" provides indication to the user that they are to position a femoral axis tool down the femoral canal in preop image 408', as shown in FIG. 1, Step 16. Digital resizing handles 462, 464, 466, and 468 allow the user to manipulate the size and shape of femoral axis annotation 472. Moving any of the handles will modify the shape of the femoral axis annotation 472 by widening or narrowing and lengthening or shortening the object based on interaction with the resizing handles. Rotation handle 470 enables the user to rotate femoral axis annotation 472. Femoral axis annotation 472 contains dotted line 474 that is displayed on screen as part of the control, which is used by the system to identify the line that runs down the middle of the femoral canal after the object has been positioned. Digital annotation 424' identifies the previously identified location of the greater trochanter, and digital circle annotation 444' identifies the previously identified location of the femoral head. Preferred implementations will try to auto-identify an appropriate starting location for the femoral axis tool using a combination of image recognition and known data of the greater trochanter 424' and femoral head position 444', which was digitally annotated in a previous step.

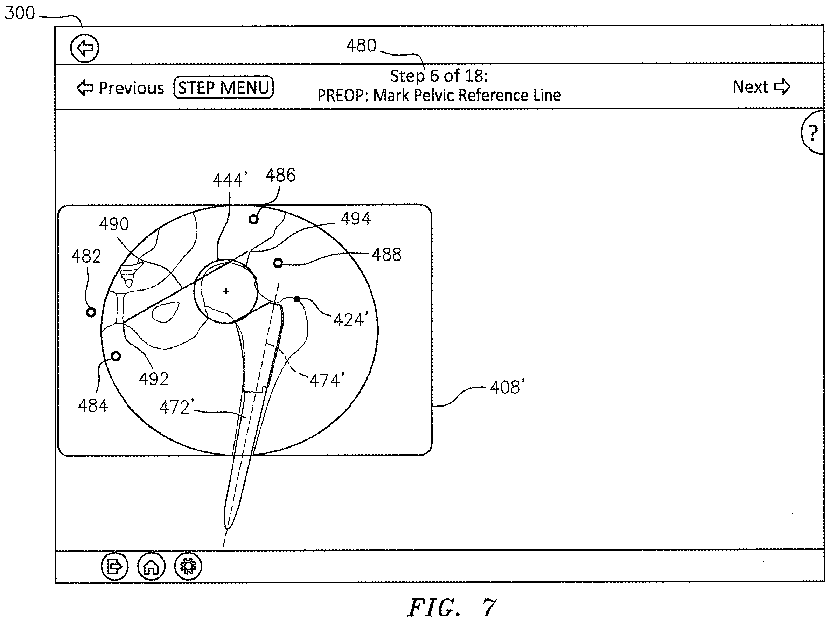

FIG. 7 shows digital screen view 300' with the same preop image 408'. Digital screen message 480 "Step 6 of 18: PREOP: Mark Pelvic Reference Line" provides indication to the user that they are to position the pelvic reference line across two identifiable anatomic points on the pelvis in preop image 408', as shown in FIG. 1A, Step 18. Digital control handles 482, 484, 486, and 488 allow the user to modify the placement of digital line 490 to accomplish this. In this image, digital line 490 has been positioned by the user so that endpoint 402 is positioned on an inferior point on the pubic symphysis, and endpoint 494 is positioned on an identifiable bony prominence on the anterior superior iliac spine. Both endpoints are readily identifiable, so that the pelvic line can be replicated in the intraop image after the hip prosthetic trial system is inserted. Digital annotation 424' identifies the previously identified location of the greater trochanter, and digital circle annotation 444' identifies the previously identified location of the femoral head. Femoral axis tool 472' with centerline 474', positioned in a previous step, is additionally shown superimposed on the preop image 408'.

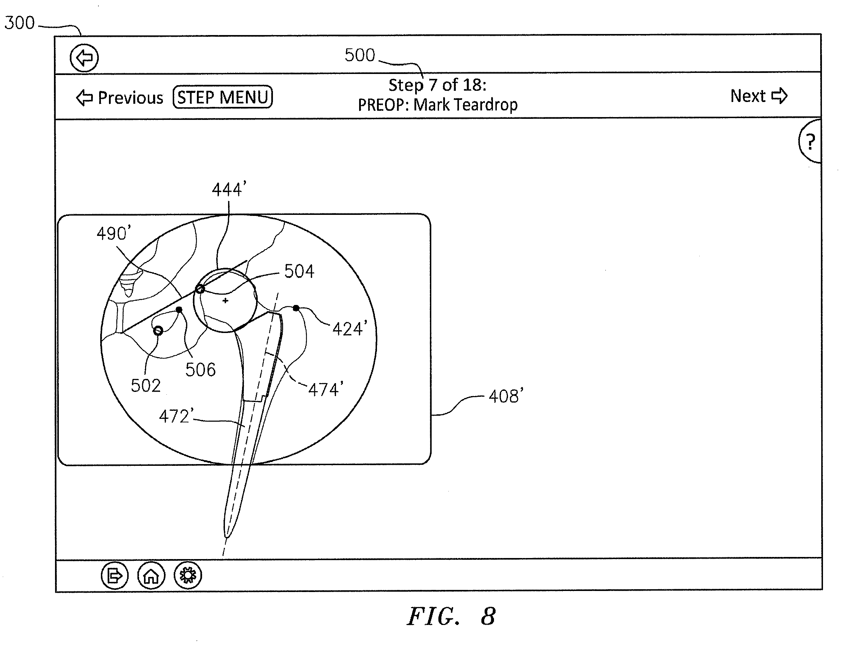

FIG. 8 shows digital screen view 300' with the same preop image 408'. Digital screen message 500 "Step 7 of 18: PREOP: Mark Teardrop" provides indication to the user that they are to position a digital annotation on the pelvic teardrop anatomy in preop image 408', as shown in FIG. 1A, Step 20. Digital control handles 502 and 504 allow the user to modify the placement of digital point 506 which is placed on the pelvic teardrop. Previously annotations, including digital annotations 424', 444', 472' and 490' are readily observable on preop image 408'.

The method continues in Step 22, FIG. 1A, in which the User Input Module 108, FIG. 2 prompts the user as to whether they would like to continue with an optional module that provides guidance for acetabular component positioning, including analysis of anteversion and inclination angles. If the user continues with this analysis, they enter a separate module in step 24 for cup analysis before continuing with image acquisition of the intraop image. If they wish to skip this analysis, they continue directly with intraop image acquisition.

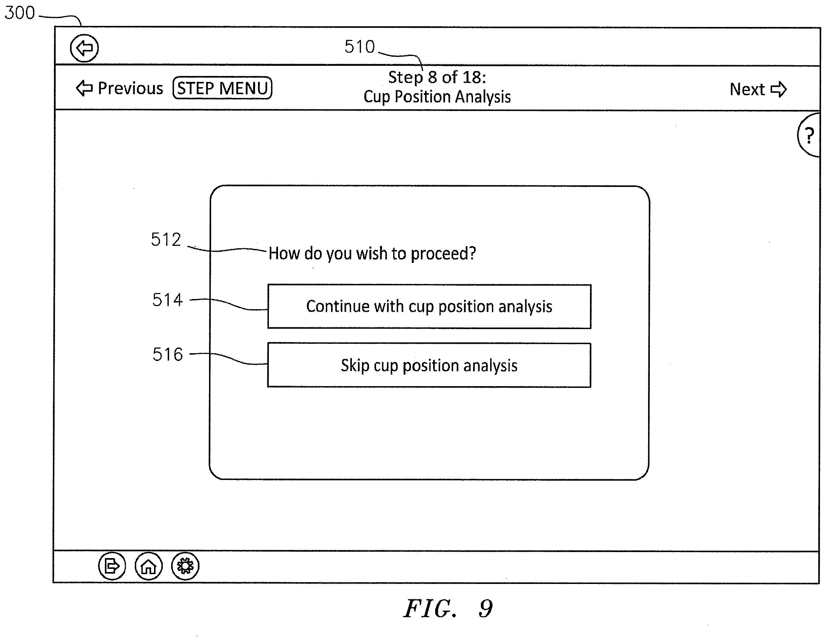

FIG. 9 implements Step 22, FIG. 1A, in which the system asks the user if they want to analyse the acetabular component placement. Digital screen message 510 "Step 8 of 18: Cup Position Analysis" informs the user that they can now commence cup position analysis, if desired. Digital screen message 512 asks the user "How do you wish to proceed?". If the user responds by selecting button 514 "Continue with cup position analysis", the system will enter a separate cup analysis system (Step 24, FIG. 1) before ultimately returning to continue with Step 26, FIG. 1. If the user responds by selecting button 514 "Skip cup position analysis", the system will skip cup analysis and immediately continue with Step 26, FIG. 1A.

In Step 26, FIG. 1A, the Image Capture Module 110, FIG. 2, guides the process of acquiring an intraop image. The intraop image is acquired after both the acetabular component prosthetic and the femoral prosthetic trial component has been put in place. The incision has not been closed and the final prosthetic not inserted, providing the surgeon with the opportunity to modify their implant selection, or modular implant option, based on the OneTrial™ system analysis.

In a preferred construction, the intraop image can be obtained by using a direct connection to a fluoroscopic c-arm or similar radiographic system. The c-arm can be connected to the computing system via an output cable that takes the fluoroscopic output as either an analog or digital signal, and inputs it into the system. Alternatively, the computing device may have an on-board or connected digital camera that can be used to take a picture of the c-arm screen. While not ideal due to operational challenges of working in an operating room during surgery, the system may also function by taking an intraop image from the system from an uploaded file or via a DICOM connection.

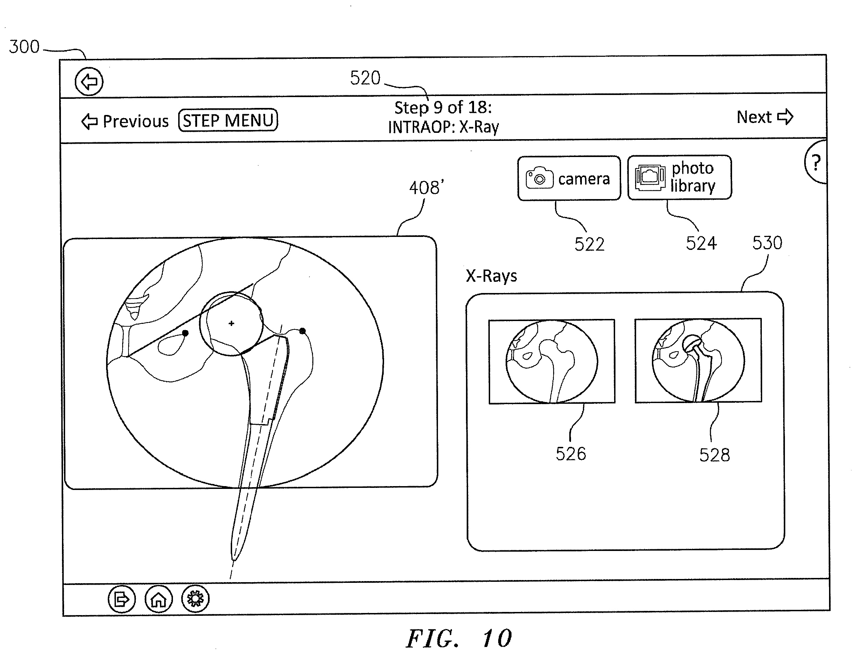

FIG. 10 implements Step 26, FIG. 1A, by providing the ability for a user to acquire an intraop image. Digital screen message 520 "Step 9 of 18: INTRAOP: X-Ray" informs the user what they are doing in this step. Touching digital "camera" button 522 commences the process of using the digital camera, connected to the computing device, to take a picture of the fluoroscopic viewing screen to acquire the intraop image. Alternatively, the user can press digital "photo library" or "digital import" button 524 to directly import an image from the fluoroscopic system. To use this direct import functionality, the computing device must be networked or connected to the fluoroscopy or radiographic unit. As an additional alternative, an uploaded image such as image 526 or 528 within digital frame 530 can be selected and used as the intraop image. Preop image 408', with digital annotations previously marked, continues to be displayed.

After acquiring the intraop image, a series of annotation steps are performed on the image in steps 28, 30, 32, 34 and 36, FIG. 1B, that are similar in nature to the previous annotations performed on the preop image.

In Step 28, FIG. 1B, the Digital Annotation Module 114, FIG. 2, guides the placement of a digital point annotation that identifies the greater trochanter on the femur. The placement of this point should match the placement of the digital point placed on the preop image in Step 12, FIG. 1A.

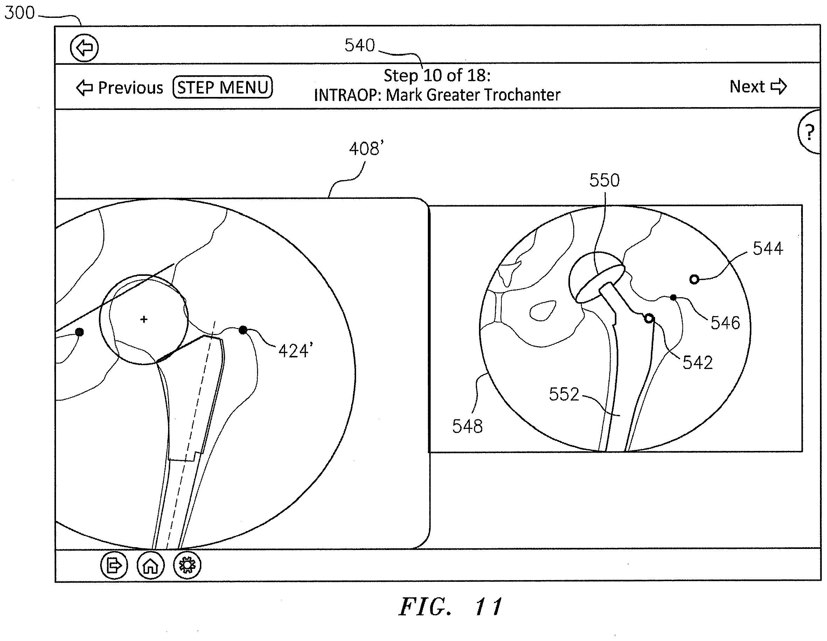

FIG. 11 shows the same digital screen view 300' with the same preop image 408' displayed on the left side of the screen and the intraop image 548 on the right side of the screen. Acetabular component 550 and femoral trial prosthetic 552 can be seen within intraop image 548. Digital screen message 540 "Step 10 of 18: INTRAOP: Mark Greater Trochanter" provides indication to the user that they are adding a digital annotation to the greater trochanter in intraop image 548, as shown in FIG. 1B, Step 28, to match the preop annotation placement performed in Step 12, FIG. 1A. The display of preop image 408' with digital annotation 424' identifying the greater trochanter in the preop image provides on-screen guidance to the user so that they can effectively match the point. Digital handles 542 and 544 allow the user to manipulate the location of digital point annotation 546 in intraop image 548, so that it can be placed on the greater trochanter in a similar position to how it was placed on preop image 408'.

In Step 30, FIG. 1B, the Digital Annotation Module 114, FIG. 2, guides the placement of a digital circle around the acetabular component. Along with the placement of the digital circle, User Input Module 108, FIG. 2 guides the user in entering the size of the acetabular component size in this step. Using this annotation and the inputted size, the Image Scaling and Alignment Module 112, FIG. 2 scales the intraop image in this step. This information will also be used later in Step 36, FIG. 1B to scale the preop image.

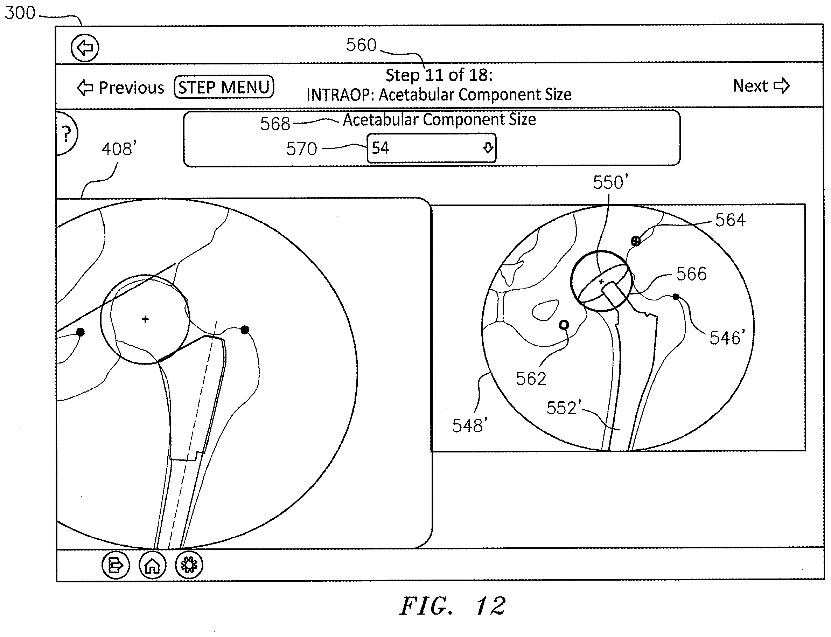

FIG. 12 shows an implementation of Step 30, FIG. 1B, in digital screen view 300' with preop image 408' on the left side of the screen and intraop image 548' on the right. Digital screen message 560 "Step 11 of 18: INTRAOP: Acetabular Component Size" guides the user to understand the functionality in this image. Within intraop image 548', digital movement handle 562 and sizing handle 564, respectively, enables the user to manipulate the location of the digital circle annotation 566 so that it precisely encapsulates the acetabular component prosthetic 550'. Digital screen message 568 "Acetabular Component Size" directs the user to input the size of the acetabular component into numerical drop down control 570. The size of the acetabular component is known because it has been open and inserted into the surgical area during surgery.

In Step 32, FIG. 1B, the Digital Annotation Module 114, FIG. 2, guides the placement of a pelvic reference line annotation on the intraop image, connecting the identical pelvic anatomic points as the line drawn on the preop image in Step 18 earlier.

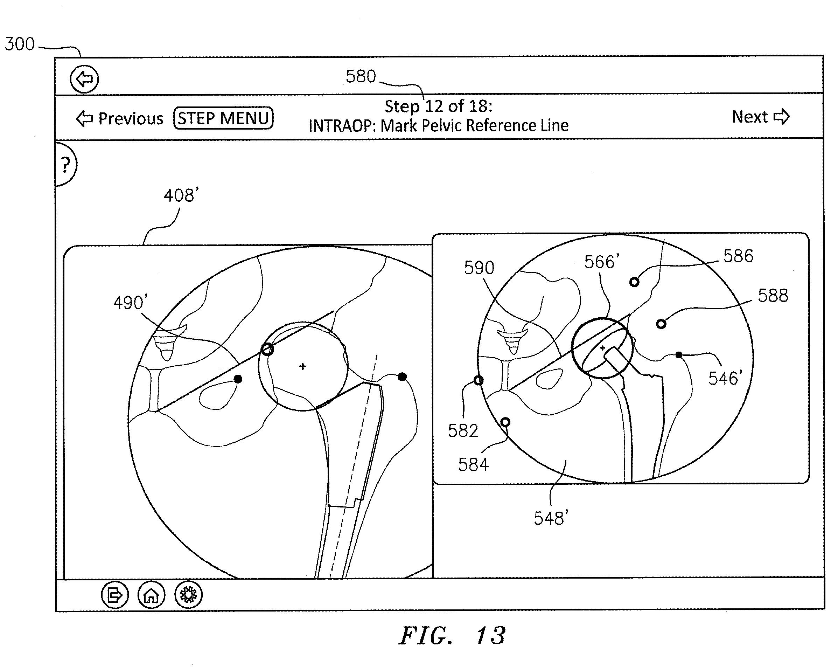

FIG. 13 shows an implementation of Step 32, FIG. 1B, in digital screen view 300' with preop image 408' on the left side of the screen and intraop image 548' on the right. Digital screen message 580 "Step 12 of 18: INTRAOP: Mark Pelvic Reference Line" provides indication to the user that they are to position the pelvic reference line across the pelvis in intraop image 548' so that it connects the identical identifiable points connected by pelvic reference line 490' in preop image 408'. Digital control handles 582, 584, 586, and 588 allow the user to modify the placement of digital pelvic reference line 590. The digital line 590 has been positioned so that the endpoints are an inferior point on the pubic symphysis and an identifiable bony prominence on the anterior superior iliac spine, consistent with the endpoints used in the preop pelvic reference line draw in Step 18, FIG. 1A. Digital annotation 546' identifies the previously identified location of the greater trochanter in intraop image 548', and previously positioned digital circle annotation 566', is also visible in intraop image 548'.

In Step 34, FIG. 1B, the final step of marking annotations on the intraop image, Digital Annotation Module 114, FIG. 2, guides the placement of a digital annotation point on the pelvic teardrop in the intraop image. This point placement should match the point that was positioned on the preop image in Step 20.

In a preferred implementation of Step 34, FIG. 1B, the point is initially auto-positioned so that it maintains the same spatial relationship, relative to the endpoints of the pelvic reference line in the intraop image, as the teardrop annotation point placed in the preop image. The initial placement of the auto-positioned teardrop annotation, relative to the pelvic teardrop anatomy, should closely match the teardrop annotation placement relative to teardrop anatomy in the preop image before any user-guided change in position are made. Any need to significantly modify the position of this teardrop annotation so that its position relative to pelvic anatomy is consistent between the preop and intraop images may indicate an error or problem in use of the system. Such an error may include a pelvic reference line that was drawn between different pelvic reference points or else differences in the orientation of the c-arm or body positioning between the preop and intraop images. In the case when the "preop image" used is in fact a contralateral hip image, the need to substantially move the teardrop may indicate that there is too much pelvic asymmetry to use data provided by the system. In a preferred construction, movement of the teardrop annotation by the user of more than 3 millimeters will render an on-screen message describing that results may be inaccurate due to user error or inconsistent imaging.

After concluding with placement of the teardrop annotation, the system continues with orienting and scaling the images, and then guiding the user to place a series of templates on the images.

In Step 36, FIG. 1B, the Image Scaling and Alignment Module 112, FIG. 2, scales the preop image and additionally aligns it so that pelvic anatomy is consistent between the preop and intraop images. The module first determines the length in pixels between the two end points in the intraop images, and resizes the preop image so that the length in pixels in the preop image is the same as the intraop image. After doing this, the system can use the same scaling information determined from Step 30, FIG. 1B, to apply absolute scaling to the preop image. Next, the system aligns the preop image so that the pelvic reference lines in the preop and intraop images are aligned consistently relative to one another, thereby ensuring that the preop and intraop images are scaled and aligned similarly based on pelvic anatomy.

In Step 38, FIG. 1B, User Input Module 108, FIG. 2, guides the user in identifying the acetabular component being used during surgery, and Templating Module 116, FIG. 2, positions an acetabular component template on the intraop image. This identifies the center of rotation by position the acetabular cup in the intraop image using a digital template or alternative digital annotation with the identified center of rotation. In a preferred implementation, the system auto-recognizes the acetabular cup in the intraop image and places a digital template directly on top of it, with the user able to adjust the placement of the template if required. The digital template may be selected based on the size of the acetabular component inserted during surgery.

Alternative constructions may instead make use of other representative digital annotations that identify the center of rotation, such as a semicircle, or otherwise a digital line drawn by the system or user to identify the base of the acetabular component. In this construction, the center of rotation corresponds to the midpoint of the digital line. As an alternative to auto-identification of the cup, the system may simply direct the user to place the template or surrogate annotation directly on top of the acetabular implant.

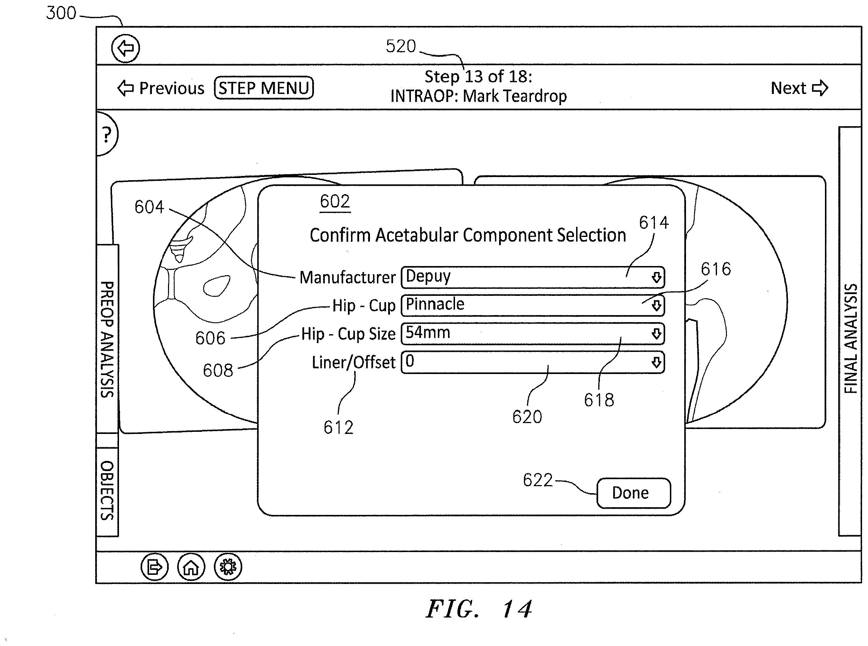

FIG. 14 shows the selection of an acetabular component template in Step 38, FIG. 1B. The surgeon and supporting health care professionals are aware of what acetabular component prosthetic is being used in surgery and can select that specific prosthetic in the system. Within the popup form 602, digital screen text captions 604 "Manufacturer", 606 "Hip - Cup", 608 "Hip - Cup Size" and 610 "Liner / Offset" provide guidance to the user to make selections in digital drop down selection objects 614, 616, 618 and 620 respectively. In this figure, the acetabular component being used is specified as "Depuy Pinnacle Size: 54 mm Liner/Offset: 0".

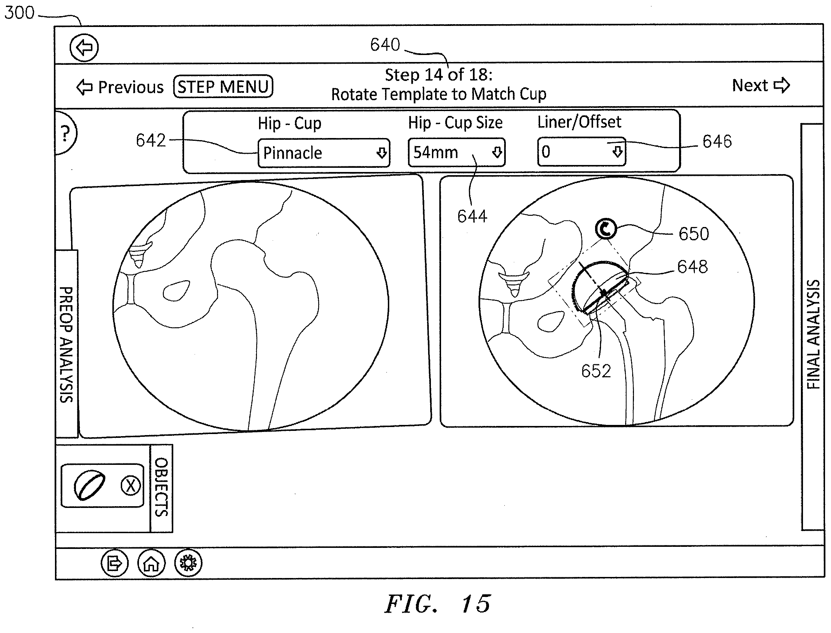

FIG. 15 shows the placement of the actual acetabular component template 648 with center of rotation 652 on the screen. Rotation handle 650 allows the user to rotate the template 648 so that it precisely overlays the acetabular component seen in the fluoroscopy image. Drop down selection objects 642, 644, and 646 allow the user to view the hip model, cup size, and liner selection chosen, as well as change the selection if an error was made in the initial selection. The digital message 640 "Step 14 of 18: Rotate Template to Match Cup" directs the user to understand what is being performed in this step.

In step 40, Templating Module 116, FIG. 2, guides the placement of a prosthetic femoral stem template, or other representative annotation, in the intraop image. In a preferred implementation, User Input Module 108 will guide the user in selecting the known manufacturer, model, and size of the femoral implant that has been surgically implanted, and will then precisely position the template directly over the actual implant in the intraop image. Some implementations of the system may also auto-recognize the femoral stem and attempt to auto-position the template. In a preferred implementation, the femoral template will be spatially connected to the acetabular template at their respective center of rotation points, because it is known that the femoral and acetabular implants are locked together in the intraoperative image. See also centers of rotation 33, 35 in FIG. 1B, co-located centers of rotation 2434i in FIG. 74, and centers of rotation 234ii and 2435ii in FIGS. 75-76 of U.S. priority Application No. 14/974,225, filed 18 December 2015 and published as U.S. 2016/ 0100909A1.

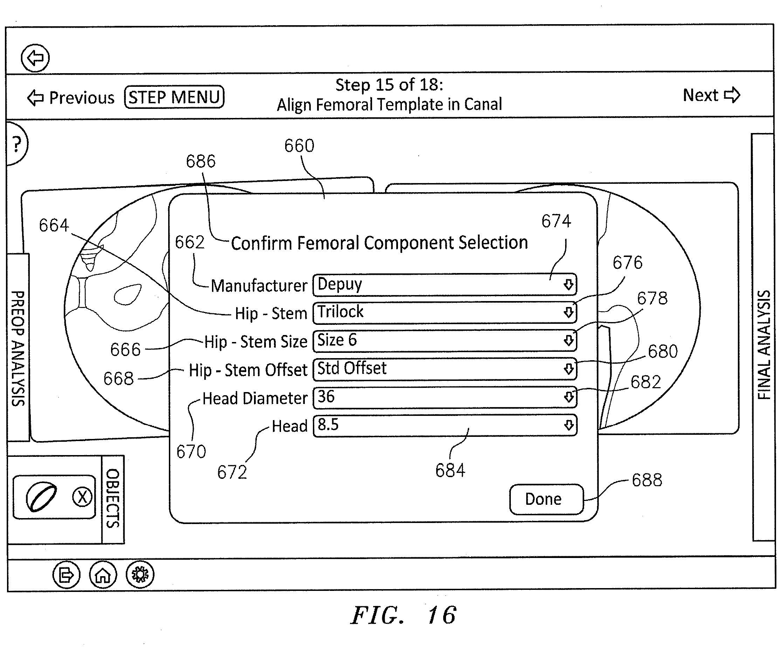

FIG. 16 shows the selection of a femoral component template in Step 40, FIG. 1B. The surgeon and supporting health care professionals are aware of what femoral trial prosthetic is being used in surgery and can select that specific prosthetic in the system. Within the popup form 660, digital screen text 686 "Confirm Femoral Component Selection" directs the user action while digital screen text captions 662 "Manufacturer", 664 "Hip - Stem", 666 "Hip - Stem Size", 668 "Hip - Stem Offset", 670 "Head Diameter" and 672 "Head" provide guidance to the user to make selections in digital drop down selection objects 674, 676, 678, 680, 682 and 684 respectively. In this figure, the femoral component used is specified as a "Depuy Trilock Size 6 Std Offset with a 36mm head diameter and an +8.5 head".

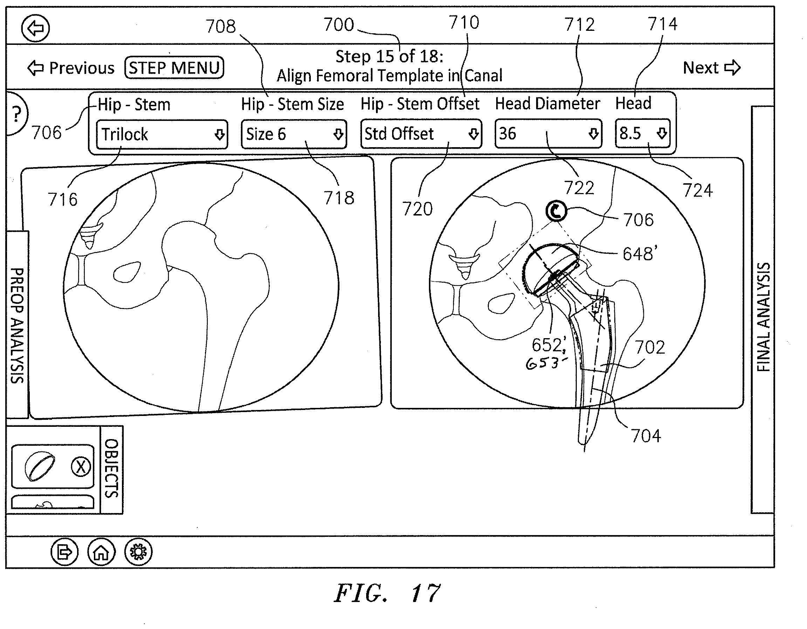

FIG. 17 shows the placement of the actual femoral component template 702 with longitudinal axis 704 and center of rotation 653', connected to the acetabular component template 648' at the co-located center of rotation 652' on the digital screen. Rotation handle 706 allows the user to rotate the femoral template 702 so that it precisely overlays the acetabular component seen in the fluoroscopy image. Because the centers of rotation 652', 653' of the acetabular and femoral components are connected intraoperatively in this implementation and are therefore co-located, the femoral template 702 can be rotated but is not movable. Drop down selection objects 716, 718, 720, 722 and 724 allow the user to view and update the hip stem model, size, and offset, as well as the head diameter and head selection that matches the trial prosthetic positioned in the body during surgery. Digital message 700 "Step 15 of 18: Align Femoral Template in Canal" provides step guidance to the user.

In Step 42, FIG. 1B, Image Scaling and Alignment Module 112, FIG. 2, positions a 'cutout' (exact copy) of the femur from the intraop image to create a digital overlay of the femur on the preop image. The system does this by connecting the preop image and the intraop 'cutout' of the femur based on the location of the greater trochanter annotations identified in Steps 12 and 28. The system provides the user with the ability to rotate the femoral overlay image around the greater trochanter point. This enables the system to precisely align the preop and intraop femurs, and to calculate any differences in preop and intraop femoral position relative to the pelvic anatomy.

FIG. 18 shows the placement of the intraop femoral 'cutout' 740 by connecting the greater trochanter annotation 744 in this intraop cutout to the placement of the preop greater trochanter annotation, also identified by 744. The user uses rotation navigation handle 742 to manipulate the cutout so that the femurs in the preop image and intraop cutout are precisely aligned. Performing this enables the system to calculate and account for any differences in femoral positioning in the preop and intraop images relative to the pelvis. Control object 746 adjusts the transparency of the femoral cutout 740, which can be helpful to the user when rotating the cutout image to match femoral alignment.

In Step 44, the system automatically places the femoral stem template on the preop image by copying the position of this template in the intraop image relative to the greater trochanter annotation on the femur. To do this, Digital Annotation Module 114 and Templating Module 116, FIG. 2, provide the positioning information of the digital annotation on the greater trochanter and the femoral template in the intraop image. Calculation Module 118, FIG. 2, uses this information to calculate the vector between these two objects. Additionally, Calculation Module 118 accounts for any difference in femoral positioning that was identified in Step 42, FIG. 1B, in which the intraop femoral cutout was overlaid and positioned on the preop image, and adjusts the vector to account for this difference. The calculated vector is used by Templating Module 116, FIG. 2, to position a copy of the intraop femoral template on the preop image. Specifically, Templating Module 116 uses the calculated vector to position the template in the preop image relative to the greater trochanter annotation on the femur in the preop image. This technique maintains the vector between the femoral template position and the digital annotation on the greater trochanter (an identifiable femoral point), while also adjusting for any differences in femoral position between the preop and intraop images relative to the pelvis.

In Step 46, FIG. 1B, the system automatically places the acetabular component template on the preop image by copying the position of this template in the intraop image relative to the pelvic teardrop annotation. To do this, Digital Annotation Module 114 and Templating Module 116, FIG. 2, provide the positioning information of the digital annotation on the pelvic teardrop and the acetabular component template in the intraop image. Calculation Module 118, FIG. 2, uses this information to calculate the vector between these two objects. The calculated vector is used by Templating Module 116 to position a copy of the intraop acetabular component template on the preop image. Effectively, this maintains the vector between the acetabular component template position and the digital annotation previously placed on the pelvic teardrop (an identifiable pelvic point).

The process of overlaying templates on the intraop image enables the system to precisely calculate where the prosthetics were placed intraoperatively in relation to the pelvis and femur, and to use this information when propagating data to the preop image in Steps 44 and 46, FIG. 1B. Any medialization or lateralization of the acetabular component will be reflected in the template positioning in Step 46, FIG. 1B, on the preop image. Similarly, any varus or valgus change in intraoperative positioning of the femoral stem will also be reflected in the template positioning in Step 44, FIG. 1B, on the preop image. This process of using intraoperative data to place templates on a preoperative image transforms an estimation process to one that can be used to precisely analyze intraoperative leg length and offset.

In Step 48, FIG. 1B, Templating Module 116, FIG. 2, calculates leg length and offset changes using the digital templates that were placed in the preop image in Steps 44 and 46, FIG. 1B. To do this, the system analyses the difference between the acetabular cup template center of rotation and the femoral stem template center of rotation. Leg length is calculated as the scale distance between these points along the longitudinal axis of the femur. This axis is generally identifiable by a straight line running down the center of the femoral template. Offset is calculated as the distance between these points along the axis perpendicular to the longitudinal axis of the femur. The use of intraoperative data to guide template placement in the preoperative image enables offset and leg length calculations that are vastly more accurate than the traditional preoperative 'estimation' of these parameters.

In Step 50, FIG. 1B, the Calculation Module 118, FIG. 2, generates an informational chart or table that calculates how leg length and offset will change if the surgeon changes the stem offset and head selections for the presently inserted femoral stem. To accomplish this, Templating Module 116 retrieves coordinate information of the shoulder point and center of rotation for the template of the inserted stem that has been stored within the template database. The module additionally retrieves shoulder coordinates and center of rotation coordinate data for all head and offset options for the inserted femoral stem from the template database. Using this information, Calculation Module 116 assumes that the shoulder point of the template remains stationary within the anatomy. This is a clinically appropriate assumption because changes to modular head selections do not require a change to the femoral stem insertion, and changes to offset options do not affect the broach that is used. Under the assumption that the position of the shoulder point for each femoral implant option is stationary and that the distal component of each option would be positioned similarly along the axis of the femur, Calculation Module 116 calculates how leg length and offset data changes for various stem offset and head combinations for the trial prosthetic being used. The system then stores a chart or table of information to display in Step 52, FIG. 1B.

In one construction according to the present invention, a table of data that can be generated in step 50 is created in relation to the schematic screen views that demonstrate the implementation of the present system and method. As shown in FIG. 16, a Depuy Trilock trial stem of size 6 has been intraoperatively positioned. The stem offset is standard, and the head size is 8.5. The head diameter, listed as 36 mm, is inconsequential with respect to the overall stem geometry because it affects neither the center of rotation nor the overall position of the stem. As shown in FIG. 19, selectable button 802 displays the calculated leg length and offset for this stem model and size, stem offset, and head diameter +8.5 option as "1mm, 2mm". This entry in the displayed data was calculated in Step 48, FIG. 1B. Under the assumption of a stationary shoulder point on the template, Calculation Module 118, FIG. 2 can calculate changes in stem size and offset for each stem variation by calculating the change in the vector between the shoulder point and the center of rotation. In the specific case of this Depuy Trilock Size 6 stem, the system generates a chart of the following data:

| [00090] | **Modular Stem Option** | [00091] | Leg Length, Offset |
|---|---|---|---|
| [00092] | Std Offset Stem, -2 Head | [00093] | -5mm, -6mm |
| [00094] | Std Offset Stem, +1.5 Head | [00095] | -3mm, -3mm |
| [00096] | Std Offset Stem, +5 Head | [00097] | -1mm, 0mm |
| [00098] | Std Offset Stem, +8.5 Head | [00099] | 1mm, 2mm |
| [000100] | Std Offset Stem, +12 Head | [000101] | 4mm, 5mm |
| [000102] | Std Offset Stem, +15.5 Head | [000103] | 6mm, 8mm |
| [000104] | High Offset Stem, -2 Head | [000105] | -5mm, 2mm |
| [000106] | High Offset Stem, +1.5 Head | [000107] | -3mm, 5mm |
| [000108] | High Offset Stem, +5 Head | [000109] | -1mm, 8mm |
| [000110] | High Offset Stem, +8.5 Head | [000111] | 1mm, 10mm |
| [000112] | High Offset Stem, +12 Head | [000113] | 3mm, 13mm |
| [000114] | High Offset Stem, +15.5 Head | [000115] | 6mm, 16mm |

All calculations in the preferred implementation have been rounded to the nearest millimeter of length or distance, because any more data granularity would not likely benefit the surgeon.

Preferred implementations of this system will render this calculated table on the screen for a selected assortment or set of various prosthetic components so that the surgeon can combine it with specific knowledge that he or she has of the current patient case. For example, if the final stem were to sit in a different position compared to the trial stem, the surgeon may wish to account for this, and having access to the entire data table of the selected assortment of prosthetics can help with this process. Alternative implementations of this system may show only a single leg length and offset value for the option that most closely approximates the surgeon's surgical target. In yet another implementation, only a portion of stem options may be rendered for the user to choose from.

In Step 52, FIG. 1B, Output Display Module 120, FIG. 2, renders all information on the screen, including calculated leg length and offset data for the inserted prosthetic system, and the informational chart calculated in Step 50 that describes how modular head and stem offset selections will affect leg length and offset data. Preferred implementations of this system may also provide preoperative analysis data that provides the surgeon with the preoperative target of the leg length and offset that they are trying to reproduce. The surgeon or health care professional can use the leg length and offset data to determine how close their results are. Then, they can use the displayed informational chart to select the optimal femoral stem offset and head options.

FIG. 19 is a screen view created in Step 52, FIG. 1B. Digital message 760 "Step 18 of 18: One Trial Analysis" informs the user that they are viewing the final analysis data in the system. Digital text 782 tells the user that the inserted acetabular component is 54 mm, and digital text 784 and 786 blocks tell the user that the stem is a Size 6 Standard Offset with a +8.5 head. Digital button 802 with text "1 mm, 2 mm" in chart 804 corresponds to this inserted stem with a Standard Offset and +8.5 head. In a preferred implementation, button 802 will initially be selected so that it is obvious to the user that this is the calculated leg length and offset for the inserted trial implant. The leg length and offset in button 802 was calculated in Step 48, FIG. 1B, by using the templates auto-positioned in the preop image. The other leg length and offset data calculations in Chart 802, FIG. 19, for other stem offset and head combinations of this size 6 stem were calculated in Stem 50, FIG. 1B. Examples of this calculated data include button 798, showing a predicted leg length and offset of "-5mm, - 6mm" for a size 6 standard offset stem with a -2 head, and button 800 shows a predicted leg length and offset of "3mm, 13mm" for a size 6 high offset stem with a +12 head.

All intraop analysis data is shown in the "Final Analysis" panel 780, FIG. 19, which is collapsible upon activation to show the underlying intraop image with the acetabular component and femoral stem templates that were positioned in Steps 38 and 40, FIG. 1B. Also within the collapsible panel, button 792 provides a toggle to change the basis of the offset calculation. Button 790 with text "Analyze New Image" provides a mechanism for the user to re-initiate the process of entering an intraop image in Step 26, FIG. 1A. This is valuable to the surgeon who, after inserting a different femoral prosthetic system based on the data in chart 804, wants to redo the analysis as a safety precaution to validate that the final leg length and offset matches the data that was calculated in Step 50, FIG. 1B. Preop image 408' is shown on the left side of the screen, with acetabular component template 794 having been placed by the system in Step 46, FIG. 1B. Acetabular template 794 corresponds to template 648' having center of rotation 652' in FIG. 17 and femoral stem template 796 corresponds to template 702 having center of rotation 653'. Centers of rotation 652', 653' are no longer co-located in FIG. 19. Of note, the template 794, FIG. 19, has been placed medially and superior to the preop position of the femoral head, demonstrating that intraoperative changes to pelvic anatomy, due to reaming, are reflected in component positioning. Also shown is femoral stem template 796 that was placed in Step 44, FIG. 1B, and greater trochanter digital annotation 744' that was placed in Step 28, FIG. 1B. Digital buttons 762 and 764 allow the user to place digital annotations on the preop and intraop images, if desired. Panel 766, collapsed in this screen view, can be opened by selecting the panel to activate it. The panel displays preoperative analysis data, including target leg length and offset that was calculated from a preoperative analysis. This allows a surgeon to compare the data provided in chart 804 to the original preoperative goals so that they can select the optimal stem offset and head for final implantation. Panel 768, also collapsed, shows digitally selectable icons to select various templates and annotations on the screen.

In alternative constructions, the system may make use of the leg length and offset data generated in Step 50, FIG. 1B, but provide it in an alternative form that does not appear as a generated chart of options. For example, the system may provide a single recommendation for a stem size, offset, and head combination based on the surgeon's desired leg length and offset parameters. In these constructions, the surgeon may have a desired leg length and offset determined preoperatively, facilitating the system's selection of a "best fit" implant selection.

Flowchart OT, FIGS. 1A-1B, provides a OneTrial™ system workflow that optimizes surgical productivity by putting preop image acquisition and preop annotation steps in the beginning of the process. This enables the system to perform Step 8 through 20 prior to acquisition of the intraop image, enabling the system user to complete these steps without the surgeon waiting for them to be performed. Nevertheless, other implementations of this system may put these steps in different orders and are within the scope of the present invention. For example, the system could guide the user to acquire both preop and intraop images before annotation and template activities are performed. Annotation steps, such as the marking of the greater trochanter, could be performed in successive steps on the preop and intraop images.

Various constructions of this system and method may provide different options to accomplish similar objectives. For example, alternative construction will alter the process of scaling and alignment of the preop and intraop images. In the preferred implementation described above, the system uses the pelvic reference line in the preop and intraop images to align both images based on aligning pelvic anatomy. Then, both images are scaled in relationship to each other by scaling one image so that the pelvic reference line length in pixels are the same in the preop and intraop images. Finally, these lines are scaled in absolute measurements based on the circle drawn around the acetabular component in Step 38, FIG. 1B. Alternative constructions of this system may use the pelvic reference line to align the pelvic anatomy, but scale each image independently. As an example, the intraop image may be scaled according to the known size of the acetabular component, whereas the preop image could be scaled in absolute terms according to the known width of the extracted femoral head. To implement this construction, the ipsilateral femoral head may be measured using calipers when extracted, and this measured distance across the width of the femoral head could be used to scale the preop image.

Alternative constructions may also obviate the need for pelvic reference lines entirely. For example, a software system residing on a digital fluoroscopy system may have been used to align and scale the images prior to image acquisition by this system. In another construction, the images may already be scaled and aligned because the surgeon took images with the patient and radiographic system in identical position. In yet another construction, the user may be prompted to manually adjust the images by changing the position and rotational alignment to match pelvic anatomy between the preop and intraop images while overlaying the two images on the computing screen.

In another modification to the preferred implementation, the alignment of femurs in the preop and intraop images in Step 42, FIG. 1B, is used to make any required adjustment to the template positioning, relative to the greater trochanter annotation, when migrating the digital implant representation of the femoral stem from the intraop to preop image. Of note, the drawing of a digital circle annotation around the femoral head in Step 14, FIG. 1A, is also used to perform this adjustment in one construction. This adjustment may be accomplished using other techniques, or otherwise can be removed from the system if the surgeon can ensure consistent femoral positioning, relative to the pelvis, when obtaining preop and intraop images. In an alternative construction, the system may make use of the femoral axis tool position in relationship to the pelvis and compare it to the femoral template positioned in the intraop image, so that any differences in femoral alignment can be identified and corrected for when the femoral template is migrated to the preoperative image. Alternative constructions that do not require this correction will remove the requirement for Steps 14, FIG. 1A and Step 42, FIG. 1B.

The greater trochanter is an ideal point to digitally annotate on the femur in Steps 12 and 28, FIGS. 1A-1B, but various alternative implementations may use an alternative representative point on the femoral anatomy, such as the lesser trochanter. For Steps 20 and 34, FIGS. 1A-1B, the teardrop is correspondingly an ideal anatomic point on the pelvis to digitally annotate for a variety of reasons, including its identifiable anatomy on radiographic images and its proximity to the acetabular component placement. However, another point on the pelvic anatomy may be used in alternative implementations, such as an identifiable point on the obturator foramen or identifiable bony anatomy on the anterior superior iliac spine. As previous described, the use of a femoral axis tool may be modified to incorporate another object that allows the identification of the longitudinal midline of the femur. The digital implant representation for the acetabular component and femoral stem may also be modified, so long as the alternative representations include center of rotation data to calculate leg length and offset differential.

Although specific features of the present invention are shown in some drawings and not in others, this is for convenience only, as each feature may be combined with any or all of the other features in accordance with the invention, which is defined by the claims. While there have been shown, described, and pointed out fundamental novel features of the invention as applied to one or more preferred embodiments thereof, it will be understood that various omissions, substitutions, and changes in the form and details of the devices illustrated, and in their operation, may be made by those skilled in the art without departing from the scope of the invention, which is defined by the claims. For example, it is expressly intended that all combinations of those elements and/or steps that perform substantially the same function, in substantially the same way, to achieve the same results be within the scope of the invention if they are covered by the claims. Substitutions of elements from one described embodiment to another are also fully intended and contemplated.

It is also to be understood that the drawings are not necessarily drawn to scale, but that they are merely conceptual in nature. Other embodiments will occur to those skilled in the art and are within the scope of the present disclosure.

## Claims

1. A method for analyzing images to optimize the restoration of orthopaedic functionality at a surgical site within a patient, the surgical site including at least a skeletal bone and an articulating bone that articulates with respect to the skeletal bone at a joint, the method comprising:
acquiring (i) a reference image including one of a preoperative image of the surgical site and a contralateral image on an opposite side of the surgical site; and (ii) an intraoperative image of the site after an implant has been implanted, the implant having first and second centers of rotation being co-located in the intraoperative image, the first center of rotation corresponding to a skeletal bone component of the implant that is affixed to the skeletal bone and the second center of rotation corresponding to an articulating bone component of the implant;
generating a first stationary point on the skeletal bone in both the intraoperative and reference images;
generating a second stationary point on the articulating bone in both the intraoperative and reference images;
identifying the location of the implant in the intraoperative image, including the position of the first and second centers of rotation;
aligning a first digital implant representation with the skeletal component of the implant; and
aligning a second digital implant representation with the articulating bone component;
copying the first digital representation and positioning it in the reference image at an equivalent location relative to the first stationary point;
copying the second digital representation and positioning it in the reference image at an equivalent location relative to the second stationary point;
determining a position of both the first and second centers of rotation relative to each other in the reference image; and
analyzing at least one of offset and length differential of the articulating bone in the intraoperative image with respect to the articulating bone in the reference image based on the difference between the first and second centers of rotation.

2. The method of claim 1, further including generating at least one of length and offset differential data for at least a portion of an alternative articulating bone component based on known dimensions of the alternative articulating bone component.

3. The method of claim 2, further including:
selecting a fixed point on the second digital implant representation;
selecting an alternative articulating bone component of the implant with known dimensions and selecting a fixed point on the alternative articulating bone component of the implant that corresponds with the fixed point on the second digital implant representation;
comparing the relative location of the fixed point on the second digital implant representation with respect to the center of rotation of the second digital implant representation and comparing the relative location of the fixed point on the alternative articulating bone component with respect to a center of rotation of the alternative articulating bone component; and
estimating changes in offset and length differential based on a difference between the comparison of the relative location of the fixed points with respect to the centers of rotation for each of the second digital implant representation and the alternative articulating bone component.

4. The method of claim 1, further including automatically providing at least a portion of an alternative articulating bone component based on known dimensions of the alternative articulating bone component to analyze at least one of offset and length differential.

5. The method of claim 1, further including generating a digital line on the skeletal bone between at least two anatomically identifiable points on both the intraoperative image and the reference image.

6. The method of claim 5, further including, responsive to a determination that the intraoperative and reference images are not orientationally aligned relative to each other, orienting the intraoperative and reference images with respect to each other based on at least the digital line on the skeletal bone.

7. The method of claim 1, further including, responsive to a determination that the intraoperative and reference images are not scaled relative to each other, scaling the intraoperative and reference images with respect to each other.

8. The method of claim 1, wherein a pelvis of the patient is selected as the skeletal bone and a femur is selected as the articulating bone, and the skeletal component of the implant is an acetabular cup and the articulating bone component includes a femoral stem.

9. The method of claim 1, wherein the first stationary point is a known location relative to an obturator foramen of the patient.

10. The method of claim 1, wherein the second stationary point on the articulating bone is a known location relative to the greater trochanter on a femur of the patient.

11. The method of claim 1, wherein the first center of rotation is measured relative to the first stationary point in the reference image according a vector calculation to determine the location of the first center of rotation in the intraoperative image.

12. The method of claim 1, further including generating a chart with a plurality of offset and length differential for an assortment of alternative implant components.

13. A system to analyze images at a surgical site within a patient, the surgical site including at least a first, skeletal bone and a second, articulating bone that articulates with the skeletal bone at a joint, the system including a memory, a user interface including a display capable of providing at least visual guidance to a user of the system, and a processor, with the processor configured to execute a program for performing the steps of the method of any one of claims 1 to 12.

14. The system of claim 13, wherein the system further comprises:
an image selection module, wherein the image selection module is capable of performing the step of acquiring (i) a reference image including one of a preoperative image of the surgical site and a contralateral image on an opposite side of the surgical site; and (ii) an intraoperative image of the site after an implant has been implanted, the implant having first and second centers of rotation being co-located in the intraoperative image, the first center of rotation corresponding to a skeletal bone component of the implant that is affixed to the skeletal bone and the second center of rotation corresponding to an articulating bone component of the implant;
a digital annotation module, wherein the digital annotation module is capable of performing the steps of generating a first stationary point on the skeletal bone in both the intraoperative and reference images, and generating a second stationary point on the articulating bone in both the intraoperative and reference images;
a templating module, wherein the templating module is capable of performing the steps of identifying the location of the implant in the intraoperative image, including the position of the first and second centers of rotation; aligning a first digital implant representation with the skeletal component of the implant; aligning a second digital implant representation with the articulating bone component; copying the first digital representation and positioning it in the reference image at an equivalent location relative to the first stationary point; copying the second digital representation and positioning it in the reference image at an equivalent location relative to the second stationary point; and
a calculation module, wherein the calculation module is capable of performing the steps of determining a position of both the first and second centers of rotation relative to each other in the reference image; and analyzing at least one of offset and length differential of the articulating bone in the intraoperative image with respect to the articulating bone in the reference image based on the difference between the first and second centers of rotation.

## Patentansprüche

1. Bildanalyseverfahren zur Optimierung der Wiederherstellung einer orthopädischen Funktion an einem Operationssitus in einem Patienten, wobei der Operationssitus mindestens einen Skelettknochen und einen artikulierenden Knochen, der bezüglich des Skelettknochens an einem Gelenk artikuliert, aufweist, wobei das Verfahren umfasst:
Erfassen (i) eines Referenzbildes, einschließlich eines von einem präoperativen Bild des Operationssitus und einem kontralateralen Bild auf einer gegenüberliegenden Seite des Operationssitus; und (ii) eines intraoperativen Bildes des Situs nach der Implantation eines Implantats, wobei das Implantat erste und zweite Drehpunkte aufweist, die gemeinsam im intraoperativen Bild angeordnet sind, wobei der erste Drehpunkt einer Skelettknochenkomponente des Implantats, die am Skelettknochen befestigt ist, entspricht und der zweite Drehpunkt einer artikulierenden Knochenkomponente des Implantats entspricht;
Erzeugen eines ersten stationären Punkts auf dem Skelettknochen sowohl im intraoperativen Bild als auch im Referenzbild;
Erzeugen eines zweiten stationären Punkts auf dem artikulierenden Knochen sowohl im intraoperativen Bild als auch im Referenzbild;
Identifizieren der Lage des Implantats im intraoperativen Bild, einschließlich der Position der ersten und zweiten Drehpunkte;
Ausrichten einer ersten digitalen Repräsentation des Implantats zur Skelettkomponente des Implantats; und
Ausrichten einer zweiten digitalen Repräsentation des Implantats zur artikulierenden Knochenkomponente;
Kopieren der ersten digitalen Repräsentation und Positionieren derselben im Bezugsbild an einer äquivalenten Stelle bezüglich des ersten stationären Punkts;
Kopieren der zweiten digitalen Repräsentation und Positionieren derselben im Bezugsbild an einer äquivalenten Stelle bezüglich des zweiten stationären Punkts;
Bestimmen einer Position sowohl des ersten als auch des zweiten Drehpunkts in Bezug aufeinander im Referenzbild; und
Analysieren mindestens einer Versatz- und/oder Längendifferenz des artikulierenden Knochens im intraoperativen Bild bezüglich des artikulierenden Knochens im Bezugsbild basierend auf dem Unterschied zwischen den ersten und zweiten Drehpunkten.

2. Verfahren nach Anspruch 1, ferner umfassend das Erzeugen mindestens von Längen- und/oder Versatzdifferenzdaten für mindestens einen Abschnitt einer alternativen artikulierenden Knochenkomponente basierend auf bekannten Abmessungen der alternativen artikulierenden Knochenkomponente.

3. Verfahren nach Anspruch 2, ferner umfassend:
Auswählen eines Fixpunkts auf der zweiten digitalen Repräsentation des Implantats;
Auswählen einer alternativen artikulierenden Knochenkomponente des Implantats mit bekannten Abmessungen und Auswählen eines Fixpunkts auf der alternativen artikulierenden Knochenkomponente des Implantats, der dem Fixpunkt auf der zweiten digitalen Repräsentation des Implantats entspricht;
Vergleichen der relativen Lage des Fixpunkts auf der zweiten digitalen Repräsentation des Implantats bezüglich des Drehpunkts der zweiten digitalen Repräsentation des Implantats und Vergleichen der relativen Lage des Fixpunkts auf der alternativen artikulierenden Knochenkomponente bezüglich eines Drehpunkts der alternativen artikulierenden Knochenkomponente; und
Schätzen von Veränderungen in der Versatz- und Längendifferenz basierend auf einem Unterschied zwischen dem Vergleich der relativen Lage der Fixpunkte bezüglich der Drehpunkte für jede der zweiten digitalen Repräsentation des Implantats und der alternativen artikulierenden Knochenkomponente.

4. Verfahren nach Anspruch 1, ferner umfassend das automatische Bereitstellen mindestens eines Abschnitts einer alternativen artikulierenden Knochenkomponente basierend auf bekannten Abmessungen der alternativen artikulierenden Knochenkomponente zur Analyse mindestens einer Versatz- und/oder Längendifferenz.

5. Verfahren nach Anspruch 1, ferner umfassend das Erzeugen einer digitalen Linie auf dem Skelettknochen zwischen mindestens zwei anatomisch identifizierbaren Punkten sowohl auf dem intraoperativen Bild als auch auf dem Referenzbild.

6. Verfahren nach Anspruch 5, ferner umfassend, als Reaktion auf eine Bestimmung, dass die intraoperativen und Referenzbilder in Bezug aufeinander nicht orientierungsmäßig ausgerichtet sind, Orientieren der intraoperativen und Referenzbilder in Bezug aufeinander basierend auf mindestens der digitalen Linie auf dem Skelettknochen.

7. Verfahren nach Anspruch 1, ferner umfassend, als Reaktion auf eine Bestimmung, dass die intraoperativen und Referenzbilder in Bezug aufeinander nicht skaliert sind, Skalieren der intraoperativen und Referenzbilder in Bezug aufeinander.

8. Verfahren nach Anspruch 1, wobei ein Becken des Patienten als Skelettknochen ausgewählt wird und ein Femur als artikulierender Knochen ausgewählt wird, und die Skelettkomponente des Implantats eine Hüftgelenkspfanne ist und die artikulierende Knochenkomponente einen Femurschaft aufweist.

9. Verfahren nach Anspruch 1, wobei der erste stationäre Punkt eine bekannte Position bezüglich eines Foramen obturatum des Patienten ist.

10. Verfahren nach Anspruch 1, wobei der zweite stationäre Punkt auf dem artikulierenden Knochen eine bekannte Position bezüglich des Trochanter major auf einem Femur des Patienten ist.

11. Verfahren nach Anspruch 1, wobei der erste Drehpunkt bezüglich des ersten stationären Punkts im Referenzbild gemäß einer Vektorberechnung zum Bestimmen der Lage des ersten Drehpunkts im intraoperativen Bild gemessen wird.

12. Verfahren nach Anspruch 1, ferner umfassend das Erzeugen einer Tabelle mit einer Vielzahl von Versatz- und Längendifferenzen für eine Auswahl an alternativen Implantatkomponenten.

13. System zum Analysieren von Bildern an einem Operationssitus in einem Patienten, wobei der Operationssitus mindestens einen ersten Skelettknochen und einen zweiten artikulierenden Knochen, der mit dem Skelettknochen an einem Gelenk artikuliert, aufweist, wobei das System einen Speicher, eine Benutzeroberfläche mit einem Anzeigebildschirm, der mindestens visuelle Anleitung für einen Benutzer des Systems bereitstellen kann, und einen Prozessor aufweist, wobei der Prozessor zum Ausführen eines Programms zur Durchführung der Schritte des Verfahrens nach einem der Ansprüche 1 bis 12 ausgelegt ist.

14. System nach Anspruch 13, wobei das System ferner umfasst:
ein Bildauswahlmodul, wobei das Bildauswahlmodul zur Durchführung des Schritts des Erfassens (i) eines Referenzbildes, einschließlich eines von einem präoperativen Bild des Operationssitus und einem kontralateralen Bild auf einer gegenüberliegenden Seite des Operationssitus; und (ii) eines intraoperativen Bildes des Situs nach der Implantation eines Implantats, wobei das Implantat erste und zweite Drehpunkte aufweist, die gemeinsam im intraoperativen Bild angeordnet sind, wobei der erste Drehpunkt einer Skelettknochenkomponente des Implantats, die am Skelettknochen befestigt ist, entspricht und der zweite Drehpunkt einer artikulierenden Knochenkomponente des Implantats entspricht, befähigt ist;
ein digitales Annotationsmodul, wobei das digitale Annotationsmodul zur Durchführung der Schritte des Erzeugens eines ersten stationären Punkts sowohl auf dem Skelettknochen im intraoperativen Bild als auch im Referenzbild und des Erzeugens eines zweiten stationären Punkts auf dem artikulierenden Knochen sowohl im intraoperativen Bild als auch im Referenzbild befähigt ist;
ein Vorlagenmodul, wobei das Vorlagenmodul zur Durchführung der Schritte des Identifizierens der Lage des Implantats im intraoperativen Bild, einschließlich der Position der ersten und zweiten Drehpunkte; des Ausrichtens einer ersten digitalen Repräsentation des Implantats zur Skelettkomponente des Implantats; des Ausrichtens einer zweiten digitalen Repräsentation des Implantats zur artikulierenden Knochenkomponente; des Kopierens der ersten digitalen Repräsentation und des Positionierens derselben im Bezugsbild an einer äquivalenten Stelle bezüglich des ersten stationären Punkts; und des Kopierens der zweiten digitalen Repräsentation und des Positionierens derselben im Bezugsbild an einer äquivalenten Stelle bezüglich des zweiten stationären Punkts befähigt ist; und
ein Berechnungsmodul, wobei das Berechnungsmodul zur Durchführung der Schritte des Bestimmens einer Position der ersten und zweiten Drehpunkte in Bezug aufeinander im Referenzbild; und des Analysierens mindestens einer Versatz- und/oder Längendifferenz des artikulierenden Knochens im intraoperativen Bild bezüglich des artikulierenden Knochens im Bezugsbild basierend auf dem Unterschied zwischen den ersten und zweiten Drehpunkten befähigt ist.

## Revendications

1. Procédé destiné à analyser des images pour optimiser la restauration d'une fonctionnalité orthopédique au niveau d'un site opératoire à l'intérieur du corps d'un patient, le site opératoire comportant au moins un os de squelette et un os d'articulation qui s'articule par rapport à l'os de squelette au niveau d'une articulation, le procédé comprenant :
l'acquisition (i) d'une image de référence comportant soit une image préopératoire du site opératoire, soit une image controlatérale sur un côté opposé du site opératoire, et (ii) d'une image peropératoire du site après qu'un implant a été implanté, l'implant ayant des premier et deuxième centres de rotation situés au même endroit dans l'image peropératoire, le premier centre de rotation correspondant à un composant d'os de squelette de l'implant qui est fixé à l'os de squelette et le deuxième centre de rotation correspondant à un composant d'os d'articulation de l'implant ;
la génération d'un premier point stationnaire sur l'os de squelette à la fois dans les images peropératoire et de référence ;
la génération d'un deuxième point stationnaire sur l'os d'articulation à la fois dans les images peropératoire et de référence ;
l'identification de l'emplacement de l'implant dans l'image peropératoire, y compris de la position des premier et deuxième centres de rotation ;
l'alignement d'une première représentation numérique d'implant avec le composant de squelette de l'implant ; et
l'alignement d'une deuxième représentation numérique d'implant avec le composant d'os d'articulation ;
la copie de la première représentation numérique et son positionnement dans l'image de référence à un emplacement équivalent par rapport au premier point stationnaire ;
la copie de la deuxième représentation numérique et son positionnement dans l'image de référence à un emplacement équivalent par rapport au deuxième point stationnaire ;
la détermination d'une position des premier et deuxième centres de rotation l'un par rapport à l'autre dans l'image de référence ; et
l'analyse d'un différentiel de décalage et/ou de longueur de l'os d'articulation dans l'image peropératoire par rapport à l'os d'articulation dans l'image de référence sur la base de la différence entre les premier et deuxième centres de rotation.

2. Procédé de la revendication 1, comportant en outre la génération de données de différentiel de longueur et/ou de décalage pour au moins une partie d'un composant d'os d'articulation alternatif sur la base de dimensions connues du composant d'os d'articulation alternatif.

3. Procédé de la revendication 2, comportant en outre :
la sélection d'un point fixe sur la deuxième représentation numérique d'implant ;
la sélection d'un composant d'os d'articulation alternatif de l'implant avec des dimensions connues et la sélection d'un point fixe sur le composant d'os d'articulation alternatif de l'implant qui correspond au point fixe sur la deuxième représentation numérique d'implant ;
la comparaison de l'emplacement relatif du point fixe sur la deuxième représentation numérique d'implant par rapport au centre de rotation de la deuxième représentation numérique d'implant et la comparaison de l'emplacement relatif du point fixe sur le composant d'os d'articulation alternatif par rapport à un centre de rotation du composant d'os d'articulation alternatif ; et
l'estimation de changements dans le différentiel de décalage et de longueur sur la base d'une différence entre la comparaison de l'emplacement relatif des points fixes par rapport aux centres de rotation à la fois pour la deuxième représentation numérique d'implant et le composant d'os d'articulation alternatif.

4. Procédé de la revendication 1, comportant en outre la fourniture automatique d'au moins une partie d'un composant d'os d'articulation alternatif sur la base de dimensions connues du composant d'os d'articulation alternatif pour analyser un différentiel de décalage et/ou de longueur.

5. Procédé de la revendication 1, comportant en outre la génération d'une ligne numérique sur l'os de squelette entre au moins deux points identifiables anatomiquement à la fois sur l'image peropératoire et l'image de référence.

6. Procédé de la revendication 5, comportant en outre, en réponse à une détermination que les images peropératoire et de référence ne sont pas alignées en orientation l'une par rapport à l'autre, l'orientation des images peropératoire et de référence l'une par rapport à l'autre au moins sur la base de la ligne numérique sur l'os de squelette.

7. Procédé de la revendication 1, comportant en outre, en réponse à une détermination que les images peropératoire et de référence ne sont pas à l'échelle l'une par rapport à l'autre, la mise à l'échelle des images peropératoire et de référence l'une par rapport à l'autre.

8. Procédé de la revendication 1, dans lequel un bassin du patient est sélectionné comme l'os de squelette et un fémur est sélectionné comme l'os d'articulation, et le composant de squelette de l'implant est une cupule acétabulaire et le composant d'os d'articulation comporte une tige fémorale.

9. Procédé de la revendication 1, dans lequel le premier point stationnaire est un emplacement connu par rapport à un trou obturateur du patient.

10. Procédé de la revendication 1, dans lequel le deuxième point stationnaire sur l'os d'articulation est un emplacement connu par rapport au grand trochanter sur un fémur du patient.

11. Procédé de la revendication 1, dans lequel le premier centre de rotation est mesuré par rapport au premier point stationnaire dans l'image de référence en fonction d'un calcul vectoriel pour déterminer l'emplacement du premier centre de rotation dans l'image peropératoire.

12. Procédé de la revendication 1, comportant en outre la génération d'un tableau avec une pluralité de différentiels de décalage et de longueur pour un assortiment de composants d'implant alternatifs.

13. Système destiné à analyser des images au niveau d'un site opératoire à l'intérieur du corps d'un patient, le site opératoire comportant au moins un premier os, de squelette, et un deuxième os, d'articulation, qui s'articule avec l'os de squelette au niveau d'une articulation, le système comportant une mémoire, une interface utilisateur comportant un écran pouvant fournir au moins des directives visuelles à un utilisateur du système, et un processeur, le processeur étant configuré pour exécuter un programme destiné à effectuer les étapes du procédé de l'une quelconque des revendications 1 à 12.

14. Système de la revendication 13, le système comprenant en outre :
un module de sélection d'images, le module de sélection d'images pouvant effectuer l'étape d'acquisition (i) d'une image de référence comportant soit une image préopératoire du site opératoire, soit une image controlatérale sur un côté opposé du site opératoire, et (ii) d'une image peropératoire du site après qu'un implant a été implanté, l'implant ayant des premier et deuxième centres de rotation situés au même endroit dans l'image peropératoire, le premier centre de rotation correspondant à un composant d'os de squelette de l'implant qui est fixé à l'os de squelette et le deuxième centre de rotation correspondant à un composant d'os d'articulation de l'implant ;
un module d'annotation numérique, le module d'annotation numérique pouvant effectuer les étapes de génération d'un premier point stationnaire sur l'os de squelette à la fois dans les images peropératoire et de référence, et génération d'un deuxième point stationnaire sur l'os d'articulation à la fois dans les images peropératoire et de référence ;
un module de modélisation, le module de modélisation pouvant effectuer les étapes d'identification de l'emplacement de l'implant dans l'image peropératoire, y compris de la position des premier et deuxième centres de rotation, alignement d'une première représentation numérique d'implant avec le composant de squelette de l'implant, alignement d'une deuxième représentation numérique d'implant avec le composant d'os d'articulation, copie de la première représentation numérique et positionnement de celle-ci dans l'image de référence à un emplacement équivalent par rapport au premier point stationnaire, copie de la deuxième représentation numérique et positionnement de celle-ci dans l'image de référence à un emplacement équivalent par rapport au deuxième point stationnaire ; et
un module de calcul, le module de calcul pouvant effectuer les étapes de détermination d'une position des premier et deuxième centres de rotation l'un par rapport à l'autre dans l'image de référence, et analyse d'un différentiel de décalage et/ou de longueur de l'os d'articulation dans l'image peropératoire par rapport à l'os d'articulation dans l'image de référence sur la base de la différence entre les premier et deuxième centres de rotation.
